# EUROPEAN PATENT APPLICATION

(11) **EP 4 851 181 A1**
(43) Date of publication of application: **09.12.2026**
(21) Application number: 25749203.3
(22) Date of filing: 31.01.2025
(51) Int. Cl.: C07K 14/005, C12N 15/86, A61K 48/00, A61P 9/12, A61P 11/00, C12N 7/00

(54) **ADENO-ASSOCIATED VIRUS VECTOR CAPABLE OF SMOOTH MUSCLE CELL-SPECIFIC GENE DELIVERY**

(30) Priority: 01.02.2024 KR 20240015880; 21.06.2024 KR 20240081460; 04.12.2024 KR 20240178930
(71) Applicant: GLUGENE THERAPEUTIC INC., Daejeon 34109 (KR)
(72) Inventor: LEE, Moon Sue, Seoul 03722 (KR); KANG, Changmin, Seoul 03722 (KR); CHOI, Hongyoung, Seoul 03722 (KR)
(74) Representative: Graf von Stosch Patentanwaltsgesellschaft mbH
(86) International application number: PCT/KR2025/001603
(87) International publication number: WO 2025/165162

(57) **Abstract**

The present disclosure relates to an adeno-associated virus vector capable of smooth muscle cell-specific gene delivery. With significantly enhanced tropism for smooth muscle cells, the adeno-associated virus variants of the present disclosure can be advantageously applied as a gene therapeutic agent for treating smooth muscle-related diseases.

## Description

### TECHNICAL FIELD

The present application claims priority based on Korean Patent Application No. 10-2024-0015880, filed on February 1, 2024, Korean Patent Application No. 10-2024-0081460, filed on June 21, 2024, and Korean Patent Application No. 10-2024-0178930, filed on December 4, 2024, and all contents disclosed in the specifications and drawings of the applications are incorporated into the present disclosure.

The present disclosure relates to an adeno-associated virus vector capable of smooth muscle cell-specific gene delivery.

### BACKGROUND ART

Gene therapy is an innovative therapeutic method that delivers genes into a patient's cells for the purpose of treating specific diseases, and it has received a lot of attention in recent years. For the successful execution of gene therapy, an efficient and safe gene delivery system capable of selectively delivering genes to target cells is necessary. One such delivery system is the adeno-associated virus (AAV) vector, which is being widely studied. Since AAV vectors have characteristics such as non-pathogenicity, stable gene expression, the ability to selectively deliver to various cells and tissues, etc., they have established themselves as an important tool in the field of gene therapy.

Smooth muscle cells (SMCs) are found in various organs, such as blood vessels, bronchi, and the digestive tract. Diseases associated with smooth muscle cells include arteriosclerosis, hypertension, asthma, irritable bowel syndrome (IBS), pulmonary hypertension, etc. Arteriosclerosis is a disease in which cholesterol and inflammatory cells accumulate on the walls of blood vessels, causing them to narrow; the proliferation and migration of smooth muscle cells play an important pathological role. In the case of hypertension, the dysfunction of smooth muscle cells, which regulate the contraction and relaxation of blood vessels, is identified as a major cause. Asthma is a disease in which the airways narrow due to excessive contraction of airway smooth muscle cells, leading to difficulty in breathing. Irritable bowel syndrome is a disease in which symptoms such as abdominal pain, constipation, diarrhea, etc. appear due to abnormal motor control of intestinal smooth muscle cells. Most of these diseases are deeply associated with abnormal activity or dysfunction of smooth muscle cells.

Therefore, a gene delivery technology targeting smooth muscle cells can play an important role in treating these diseases. However, since most AAV vectors developed to date lack specificity for smooth muscle cells, there is a problem wherein genes are also delivered to non-target cells or tissues. This increases the likelihood of unwanted side effects and can reduce therapeutic efficacy.

To enable smooth muscle cell-specific gene delivery, strategies are being studied wherein the capsid protein of AAV vectors is modified or specific promoters are utilized. The modification of the capsid protein increases the binding affinity to receptors on the surface of smooth muscle cells, thereby increasing the efficiency of intracellular uptake. Additionally, methods utilizing specific promoters are designed to selectively express genes only in target cells. These approaches can simultaneously improve the safety and efficiency of gene therapy by enhancing specificity for smooth muscle cells.

### DISCLOSURE

### Technical Problem

The present disclosure aims to overcome the limitations of the existing technology and maximize the efficiency of gene therapy by providing a new AAV vector that enables smooth muscle cell-specific gene delivery.

### Technical Solution

The inventors of the present disclosure have conducted extensive research and discovered that the tropism for smooth muscle cells is significantly increased by modifying the capsid protein of an adeno-associated virus (preferably by point mutation, peptide insertion mutation, or both), and have completed the present disclosure.

The present disclosure provides an adeno-associated virus variant, preferably a variant of the adeno-associated virus capsid protein, capable of specifically targeting smooth muscle cells. The capsid protein of the adeno-associated virus variant may have at least one amino acid substituted with another amino acid as compared to the wild-type capsid protein, and may also include a peptide insertion sequence in the capsid protein.

The term adeno-associated virus or AAV used in the present disclosure refers to all adeno-associated viruses used in gene therapy, including their derivatives, viral subtypes, and naturally occurring and recombinant forms. Various serotypes of AAV can be used as recombinant gene delivery viruses to transduce a number of different cell types. The genomic sequences of various serotypes of AAV, as well as the sequences of natural terminal repeats (TRs), Rep proteins, and capsid subunits, are known in the art. These sequences can be found in the literature or in public databases such as GenBank. For example, refer to GenBank accession numbers NC_002077 (AAV-1) and AF063497 (AAV-1).

In an aspect of the present disclosure, the adeno-associated virus variant may be a variant derived from serotypes AAV1, AAV2, AAV3, AAV4, AAV5, AAV6, AAV7, AAV8, AAV9, AAVrh74, etc., although not being limited thereto. The term serotype refers to a subdivision of AAV that can be identified by serological or DNA sequencing methods and distinguished by its antigenic characteristics. The adeno-associated virus variant may be a variant derived from the wild-type AAV1.

In an aspect of the present disclosure, the term capsid refers to a protein that forms the outer shell of a virus, which is encoded by the cap gene present in the virus genome. The wild-type AAV genome or cap gene encodes three types of capsid proteins (VP1, VP2, and VP3).

In an aspect of the present disclosure, the variant of the adeno-associated virus capsid protein may be a variant of the AAV1 capsid protein, and one or more amino acids selected from the group consisting of the 14th, 21st, 23rd, 24th, 31st, 32nd, 35th, 36th, 37th, 38th, 39th, 42nd, 137th, 310th, 415th, 430th, 492nd, 516th, 583rd, 647th, 660th, and 688th amino acids may be replaced with another amino acid as compared to the wild-type AAV1 capsid protein (SEQ ID NO: 1). In an aspect of the present disclosure, when the variant of the adeno-associated virus capsid protein is a variant of the AAV1 capsid protein, as compared to the wild-type AAV1 capsid protein, the 14th amino acid may be replaced with threonine (T) instead of asparagine (N) (N14T), the 21st amino acid may be replaced with glutamine (Q) instead of glutamic acid (E) (E21Q), the 23rd amino acid may be replaced with arginine (R) instead of tryptophan (W) (W23R), the 24th amino acid may be replaced with lysine (K) instead of aspartic acid (D) (D24K), the 31st amino acid may be replaced with threonine (T) instead of lysine (K) (K31T), and the 32nd amino acid may be replaced with threonine (T) instead of proline (P) (P32T), the 35th amino acid may be replaced with arginine (R) instead of asparagine (N) (N35R), the 36th amino acid may be replaced with glutamic acid (E) instead of glutamine (Q) (Q36E), the 37th amino acid may be replaced with arginine (R) instead of glutamine (Q) (Q37R), the 38th amino acid may be replaced with histidine (H) instead of lysine (K) (K38H), the 39th amino acid may be replaced with lysine (K) instead of glutamine (Q) (Q39K), the 42nd amino acid may be replaced with serine (S) instead of glycine (G) (G42S), the 137th amino acid may be replaced with glutamic acid (E) instead of lysine (K) (K137E), the 310th amino acid may be replaced with arginine (R) instead of lysine (K) (K310R), the 415th amino acid may be replaced with isoleucine (I) instead of threonine (T) (T415I), the 430th amino acid may be replaced with arginine (R), cysteine (C), or glutamic acid (E) instead of serine (S) (S430R, S430C, or S430E), the 492nd amino acid may be replaced with alanine (A) instead of threonine (T) (T492A), the 516th amino acid may be replaced with threonine (T) instead of serine (S) (S516T), the 583rd amino acid may be replaced with aspartic acid (D) instead of asparagine (N) (N583D), the 647th amino acid may be replaced with alanine (A), valine (V), or tyrosine (Y) instead of isoleucine (I) (I647A, I647V, or I647Y), the 660th amino acid may be replaced with valine (V) instead of alanine (A) (A660V), and the 688th amino acid may be replaced with arginine (R) instead of glutamine (Q) (Q688R).

In an aspect of the present disclosure, the variant of the adeno-associated virus capsid protein may include S430C and I647V substitutions as compared to the wild-type AAV1 capsid protein, and the amino acid sequence of this variant is represented by SEQ ID NO: 2. In addition, the variant of the adeno-associated virus capsid protein may include S516T, N583D and A660V substitutions as compared to the wild-type AAV1 capsid protein, and the amino acid sequence of this variant is represented by SEQ ID NO: 3. In addition, the variant of the adeno-associated virus capsid protein may include K310R substitution as compared to the wild-type AAV1 capsid protein, and the amino acid sequence of this variant is represented by SEQ ID NO: 4. In addition, the variant of the adeno-associated virus capsid protein may include W23R and T415I substitutions as compared to the wild-type AAV1 capsid protein, and the amino acid sequence of this variant is represented by SEQ ID NO: 5. In addition, the variant of the adeno-associated virus capsid protein may include N14T, E21Q, D24K, K31T, P32T, N35R, Q36E, Q37R, K38H, Q39K, and G42S substitutions as compared to the wild-type AAV1 capsid protein, and the amino acid sequence of this variant is represented by SEQ ID NO: 6. In addition, the variant of the adeno-associated virus capsid protein may include Q688R substitution as compared to the wild-type AAV1 capsid protein, and the amino acid sequence of this variant is represented by SEQ ID NO: 7. In addition, the variant of the adeno-associated virus capsid protein may include a T492A substitution as compared to the wild-type AAV1 capsid protein, and the amino acid sequence of this variant is represented by SEQ ID NO: 8. In addition, the variant of the adeno-associated virus capsid protein may include a K137E substitution as compared to the wild-type AAV1 capsid protein, and the amino acid sequence of this variant is represented by SEQ ID NO: 9.

In an aspect of the present disclosure, the substitution at the 430th amino acid position may include the replacement of serine with arginine (R) or glutamic acid (E) (S430R, S430E), in addition to serine being replaced with cysteine, and the amino acid sequences of these variants are represented by SEQ ID NO: 10 or 11, respectively. Also, the substitution of the 647th amino acid may be isoleucine being replaced with alanine (A) or tyrosine (Y) (I647A, I647Y) in addition to isoleucine being replaced with valine, and the amino acid sequences of these variants are represented by SEQ ID NO: 12 or 13, respectively.

In an aspect of the present disclosure, the variant of the adeno-associated virus capsid protein may be a variant of the AAV capsid protein of a serotype other than AAV1, such as AAV2, AAV3, AAV4, AAV5, AAV6, AAV7, AAV8, AAV9, or AAVrh74. In such cases, amino acid substitution may occur at the amino acid positions of other serotypes corresponding to each substitution position of the wild-type AAV1.

In an aspect of the present disclosure, the peptide insertion sequence included in the capsid protein of the adeno-associated virus variant may be a peptide insertion sequence composed of X₁X₂X₃X₄X₅X₆X₇ (Formula I). By including the specific peptide insertion sequence in the capsid protein of the adeno-associated virus variant, along with amino acid substitution at a specific position of the adeno-associated virus variant's capsid protein, the affinity of the adeno-associated virus variant for surface receptors of smooth muscle cells may be increased significantly.

In an aspect of the present disclosure, the peptide insertion sequence composed of X₁X₂X₃X₄X₅X₆X₇ (Formula I) may be inserted into the loop region of the wild-type adeno-associated virus, preferably wild-type AAV1, preferably the GH loop region. Preferably, the peptide insertion sequence may be inserted between two adjacent amino acids among the 580th to 600th amino acids, more preferably between the 588th and 589th amino acids, of the wild-type AAV1. Alternatively, the peptide insertion sequence may be inserted at an amino acid position of another serotype capsid protein corresponding to the insertion site of the wild-type AAV1.

In an aspect of the present disclosure, the amino acid X₁ may be any one selected from the group consisting of S, L, A, P, N, V, D, R, E, Q, and T. The amino acid X₂ may be any one selected from the group consisting of S, N, G, R, T, A, P, E, V, Q, and K. The amino acid X₃ may be any one selected from the group consisting of T, E, S, N, V, P, R, A, G, K, and D. The amino acid X₄ may be any one selected from the group consisting of S, T, P, K, D, Q, Y, A, V, L, and N. The amino acid X₅ may be any one selected from the group consisting of P, S, T, D, G, A, N, L, K, R, and I. The amino acid X₆ may be any one selected from the group consisting of L, Q, T, V, N, G, P, S, A, K, and D. And, the amino acid X₇ may be any one selected from the group consisting of S, T, P, G, Q, K, A, R, V, H, and E.

In an aspect of the present disclosure, Chemical Formula I may include at least one of the amino acids specified for X₁ to X₇, or all of them.

In an aspect of the present disclosure, in Chemical Formula I, (a) X₁ may be any one selected from the group consisting of A, R, N, D, L, P, and S, X₇ may be any one selected from the group consisting of A, Q, G, P, S, T, and V, and X₂, X₃, X₄, X₅, and X₆ may independently be any amino acid;

(b) X₅ may be any one selected from the group consisting of A, N, G, I, P, S, and T, X₆ may be any one selected from the group consisting of R, N, Q, G, L, T, and V, and X₁, X₂, X₃, X₄, and X₇ may independently be any amino acid; or

(c) X₂ may be any one selected from the group consisting of R, N, Q, G, P, S, and T, X₃ may be any one selected from the group consisting of N, D, E, P, S, T, and V, and X₁, X₄, X₅, X₆, and X₇ may independently be any amino acid.

In an aspect of the present disclosure, the insertion sequence may be any one of the insertion sequences listed in Table 1 below. Preferably, the insertion sequence may be any one selected from the group consisting of SGVSILG (SEQ ID NO: 151), SPNSAGV (SEQ ID NO: 152), and SQDHGRS (SEQ ID NO: 264).

In an aspect of the present disclosure, the capsid amino acid sequence of an adeno-associated virus variant including SPNSAGV as a peptide insertion sequence and without point mutation is shown as SEQ ID NO: 14. Additionally, the capsid amino acid sequence of an adeno-associated virus variant including SGVSILG as a peptide insertion sequence and without point mutation is shown as SEQ ID NO: 15. Additionally, the capsid amino acid sequence of an adeno-associated virus variant including SPNSAGV as a peptide insertion sequence and including S430C and I647V point mutations is shown as SEQ ID NO: 16. Additionally, the capsid amino acid sequence of an adeno-associated virus variant including SGVSILG as a peptide insertion sequence and including S430C and I647V point mutations is shown as SEQ ID NO: 17. The above-mentioned variants including combinations of the peptide insertion sequences and point mutations can synergistically and significantly increase the responsiveness to smooth muscle.

In an aspect of the present disclosure, the variant of the adeno-associated virus capsid protein may exhibit tropism for smooth muscle cells, preferably human smooth muscle cells. The human smooth muscle cells may be smooth muscle cells present in the digestive system (esophagus, stomach, small intestine, large intestine, rectum and anus), respiratory system (lungs, trachea, bronchi, bronchioles), urinary system (kidneys, ureters, bladder, urethra), reproductive system (vas deferens, epididymis, prostate, uterus, fallopian tubes, vagina), vascular system (arteries, veins), lymphatic vessels, eyes (iris muscles, ciliary muscles), skin (periphery of hair follicles), and other organs (bile ducts, pancreatic ducts, etc.), although not being limited thereto.

The term tropism may refer to the specificity of the AAV capsid protein present in the AAV virus particle for infecting or transducing a specific type of cell or tissue. The tropism refers to the ability of an AAV vector or virion to infect one or more specific cell types, but may also include whether the vector functions to transduce a cell into one or more specific cell types. That is, the tropism refers to the preferential introduction of the AAV vector or virion into a specific cell or tissue type and/or the expression (transcription and translation) of the sequence carried by the AAV vector or virion in the cell after a preferential interaction with the cell surface that facilitates entry into the specific cell or tissue type, e.g., in the case of a recombinant virus, the expression of a heterologous nucleotide sequence.

In addition, the present disclosure provides a nucleic acid encoding the variant of the adeno-associated virus capsid protein. The nucleic acid may encode the variant of the AAV1 capsid protein. The nucleotide sequences corresponding to the amino acid sequences of SEQ ID NOS: 1 to 17 are represented as SEQ ID NOS: 18 to 34. In the present disclosure, the nucleic acid is constructed by substituting at least one nucleotide with another nucleotide in the nucleotide sequence of the nucleic acid (cap gene) encoding the AAV1 capsid protein. The nucleic acid of the present disclosure may exist in the form of DNA, but in some cases, it may be in the form of RNA or a chimera of DNA and RNA. In addition, the nucleic acid of the present disclosure includes a complementary nucleic acid (e.g., cDNA). The nucleic acid of the present disclosure may be single-stranded or double-stranded. Preferably, it may be double-stranded.

In an aspect of the present disclosure, the nucleic acid may be operably linked with an appropriate regulatory sequence. The regulatory sequence includes a promoter sequence, a polyadenylation signal, a transcription termination sequence, an upstream regulatory domain, an internal ribosome entry site (IRES), an enhancer, etc. The promoter sequence includes an inducible promoter sequence and a constitutive promoter sequence. The regulatory sequence may be native to the AAV from which the capsid protein is derived or foreign, and may be a natural sequence or a synthetic sequence.

Furthermore, the present disclosure provides a recombinant adeno-associated virus vector containing the nucleic acid. The recombinant AAV vector is useful for introducing a gene into a target cell, and the gene introduced by the recombinant AAV vector may be strongly expressed in the target cell. The recombinant AAV vector is useful for delivering the nucleic acid of the present disclosure to cells *in vitro*, *ex vivo*, and *in vivo*, and for conferring the ability to express the variant of the AAV1 capsid protein to the cells. Additionally, the cells to which the nucleic acid of the present disclosure has been delivered are also useful for producing recombinant AAV particles. The recombinant DNA can be used to deliver or introduce the nucleic acid of the present disclosure to eukaryotic cells, preferably animal cells, and more preferably mammalian cells. In the present disclosure, the recombinant DNA can be produced by incorporating the nucleic acid of the present disclosure into DNA used as a vector. For example, a plasmid, a phage, a transposon, a cosmid, an episomal DNA, a viral genome, etc., may be used. For example, a packaging plasmid can be constructed using a nucleic acid encoding the variant of the AAV1 capsid protein of the present disclosure (cap gene) in a plasmid. The packaging plasmid may additionally include any nucleic acid sequence, such as a nucleic acid encoding a replicase (Rep) protein (rep gene). Preferably, the rep gene may include AAV2-derived Rep. In the nucleic acid sequence of the cap gene packaged in a known packaging plasmid, a recombinant AAV vector containing the nucleic acid of the present disclosure can also be constructed by substituting at least one nucleotide in the PLA2 domain coding region with another nucleotide. The packaging plasmid is not particularly limited, but examples include a packaging plasmid with a cap gene, and suitably, a packaging plasmid with both a cap gene and a rep gene.

In an aspect of the present disclosure, the AAV virus refers to a virus particle consisting of at least one AAV capsid protein and a polynucleotide rAAV vector encapsidated into the capsid. If the particle contains a heterologous polynucleotide (i.e., a polynucleotide other than the wild-type AAV genome, such as a transgene delivered to a mammalian cell), it is typically referred to as an rAAV vector particle or simply an rAAV vector. Thus, the production of a rAAV particle necessarily involves the production of rAAV, since such a vector is contained within the rAAV particle. The term packaging refers to a series of intracellular events that cause the assembly and encapsidation of the AAV particle. The AAV rep and cap genes refer to polynucleotide sequences encoding the replication and encapsidation proteins of the adeno-associated virus. The AAV rep and cap are referred to herein as the AAV packaging genes.

A helper virus for AAV refers to a virus that enables AAV to be replicated and packaged by mammalian cells. Various such helper viruses for AAV, including adenoviruses, herpesviruses, and poxviruses such as vaccinia, are known in the art. Although adenovirus type 5 of subgroup C is the most commonly used, adenoviruses include a number of different subgroups. Numerous adenoviruses of human, non-human mammalian, and avian origin are known and are available from depositary institutions such as the ATCC. Viruses of the herpes family include, for example, herpes simplex viruses (HSV) and Epstein-Barr viruses (EBV), as well as cytomegaloviruses (CMV) and pseudorabies viruses (PRV), which are also available from depositary institutions such as the ATCC. Helper virus function refers to a function encoded in the helper virus genome that allows for AAV replication and packaging. As described in the present disclosure, the helper virus function may be provided in various ways, such as by providing a helper virus or, for example, by providing a polynucleotide sequence encoding an essential function to a producer cell in a trans manner. For example, a plasmid or other expression vector containing a nucleotide sequence encoding one or more adenovirus protein may be transfected into a producer cell along with the recombinant AAV vector.

In addition, the present disclosure provides a pharmaceutical composition for preventing or treating a smooth muscle cell-related disease, which contains the recombinant adeno-associated virus vector. The smooth muscle cell-related disease includes a vascular smooth muscle cell-related disease (e.g., hypertension, aneurysm, atherosclerosis), leiomyomas, leiomyosarcomas, a smooth muscle relaxation disorder (e.g., achalasia, bladder outlet obstruction), smooth muscle hypercontractility (e.g., bronchospasm, intestinal spasm, uterine hypercontractility), congenital smooth muscle abnormalities (e.g., congenital megacolon, pulmonary hypertension), smooth muscle infections and inflammatory diseases (e.g., endometritis), and other metabolic and hereditary smooth muscle diseases (e.g., congenital leiomyomatosis, mitochondrial disease), although not being limited thereto.

In the present disclosure, the term treatment refers to any type of intervention or process performed on a subject or the administration of an active agent to the subject for the purpose of reversing, alleviating, improving, inhibiting, delaying, or preventing the progression, development, severity, or recurrence of a disease-related syndrome, complication, symptom, or biochemical sign. The treatment may be performed on a subject with the disease or on a subject without the disease (e.g., for prevention).

Additionally, the present disclosure provides a method for preventing or treating a smooth muscle cell-related disease, which includes administering a therapeutically effective amount of the pharmaceutical composition described above to a subject. The administration refers to the physical introduction of a therapeutic agent or a composition containing the therapeutic agent to the subject using any of the various methods and delivery systems known to those skilled in the art. Preferred routes of administration include intravenous, intraperitoneal, intramuscular, subcutaneous, spinal, intravitreal, or other parenteral routes of administration, for example, by injection or infusion. The parenteral administration generally refers to a method of administration other than intestinal and local administration by injection, and is not limited thereto, but includes intravenous, intraperitoneal, intramuscular, intra-arterial, intramedullary, intralymphatic, intralesional, intracapsular, intraorbital, intracardiac, intradermal, transcanal, subcutaneous, subepidermal, intravitreal, intra-articular, subcapsular, subarachnoid, intramedullary, epidural, and intrasternal injections and infusions, as well as *in vivo* electroporation.

In an aspect of the present disclosure, the therapeutically effective amount refers to an amount of a drug alone or in combination with another therapeutic agent that is effective in treating a disease or disorder (e.g., pulmonary arterial hypertension) of a subject or reducing the risk, potential, likelihood, or occurrence of the disease or disorder. The therapeutically effective amount includes an amount of a drug or therapeutic agent that provides some improvement or benefit to a subject who has or is at risk of having the disease or disorder. Accordingly, the therapeutically effective amount is an amount that reduces the risk, potential, likelihood, or occurrence of the disease or disorder, or provides some alleviation or relief, or reduces at least one indicator, or reduces at least one clinical symptom of the disease or disorder.

In an aspect of the present disclosure, the subject includes any human or non-human animal. The non-human animal includes all vertebrates, e.g., mammals and non-human primates, sheep, dogs, cattle, chickens, and non-mammals such as amphibians, reptiles, etc.

### Advantageous Effects

With significantly enhanced tropism for smooth muscle cells, an adeno-associated virus variant of the present disclosure can be advantageously used as a gene therapeutic agent for treating smooth muscle cell-related diseases.

### DESCRIPTION OF DRAWINGS

FIG. 1 shows the result of imaging the tropism, within lung tissue, of the variants screened 3 times in mouse lung smooth muscle cells using X-gal staining.
FIG. 2 shows the result of imaging the migration and delivery capabilities of wild-type AAV1 and variants AAV1.S430C, AAV1.I647V, and AAV1.p2CV into pulmonary artery smooth muscle cells using immunohistochemistry.
FIG. 3 shows the result of monitoring the movement trajectories of wild-type AAV1 and variants AAV1.S430C, AAV1.I647V, and AAV1.p2CV in pH 7.4 PBS.
FIG. 4 shows the MSD values in the trajectories of wild-type AAV1 and variants AAV1.S430C, AAV1.I647V, and AAV1.p2CV in pH 7.4 PBS depending on the length of measurement time.
FIG. 5 shows the trajectories and MSD values of variants AAV1.I647A, AAV1.I647V, AAV1.I647Y, AAV1.S430R, AAV1.S430C, and AAV1.S430E in pH 7.4 PBS.
FIG. 6 shows the result of delivering the green fluorescent protein (GFP) gene to human pulmonary artery smooth muscle cells (hPASMCs) *in vitro* using wild-type AAV1, variant AAV1.S430C, AAV1.I647V, and AAV1.p2CV, and measuring the GFP expression cell ratio and average brightness through fluorescence imaging.
FIG. 7 shows the result of delivering the human FGF12 (hFGF12) gene to a mouse model of pulmonary arterial hypertension *in vivo* via airway aerosol administration using wild-type AAV1 and the variant AAV1.p2CV, and measuring the hFGF12 mRNA expression level in pulmonary smooth muscle cells (PSMC) by RT-PCR.
FIG. 8 shows the result of delivering the hFGF12 gene to human pulmonary arterial smooth muscle cells (hPASMC) *in vitro* using AAV1-based adeno-associated virus variants in Experimental Example 14, and measuring the hFGF12 mRNA expression level by cDNA qPCR.
FIGS. 9 and 10 show the result of delivering the human FGF12 (hFGF12) gene to human pulmonary artery smooth muscle cells (hPASMC) *in vitro* using AAV1-based adeno-associated virus variants in Experimental Example 14, and measuring the mRNA expression levels of the ACTA and TAGLN genes expressed through hFGF12 by cDNA qPCR.
FIGS. 11 and 12 show the result of testing the preventive effect against pulmonary arterial hypertension (PAH) *in vivo* using AAV1-based adeno-associated virus variants in Experimental Example 15.

### BEST MODE

Hereinafter, examples, etc. will be described in detail to aid in understanding the present disclosure. However, the examples according to the present disclosure may be modified in various different forms, and the scope of the present disclosure should not be interpreted as being limited to the following examples. The examples of the present disclosure are provided to more completely explain the present disclosure to a person having ordinary skill in the art to which the present disclosure belongs.

### Experimental Example 1: Preparation of AAV1-based point mutation adeno-associated virus library

Random mutations were introduced into the wild-type AAV1 cap gene using error-prone PCR based on wild-type AAV1. During the error-prone PCR process, various concentrations of manganese(II) chloride (Sigma-Aldrich, St. Louis, MO; final concentrations of 0.05 mM, 0.15 mM, and 0.3 mM in the total PCR mixture) and dNTP ratios (2 mM ATP, 2 mM GTP, 1 mM TTP, 1 mM CTP) were used in the cap gene amplification process, resulting in random mutations in each cap gene. Each mutated cap gene was extended using Taq polymerase (Thermo Fisher Scientific, Waltham, MA). Each error-prone PCR product downstream of rep2, bounded by inverted terminal repeats (ITRs), was cloned to generate a plasmid library including AAV capsid genes with random point mutations.

A combination of the AAV1-based point mutation plasmid library, Bluescript plasmids (Stratagene, La Jolla, CA), and adenovirus helper plasmids (pHelper; Stratagene, La Jolla, CA) was suspended in 2.5 M CaCl₂ solution and combined slowly with a 2x HEPES buffer containing 280 mM NaCl, 1.5 mM Na₂HPO₄, and 50 mM HEPES at pH 7.1. Transformation was performed by adding the final mixture dropwise to HEK293T cells cultured in a 15-cm tissue culture plate. 72 hours after the transformation, the HEK293T cells were harvested and lysed through three freeze-thaw cycles, and the lysate was treated with Benzonase (Aldrich; 1 unit/mL) to remove cellular genomic contaminants. The virus vector was purified using iodixanol density gradient ultracentrifugation (OptiPrep, Alere Technologies AS, Oslo, Norway) with a Vti65.2 rotor (Beckman Coulter; 42,000 rpm). The purified AAV vector was buffer-exchanged with a 1x PBS solution containing 0.01% (v/v) Tween-20 (Sigma-Aldrich) using an Amicon tube (Merck Millipore, Billerica, MA; molecular weight cutoff: 100 kDa).

### Experimental Example 2: Screening of lung smooth muscle cell-targeting variants from AAV1-based point mutation adeno-associated virus library

The recombinant AAV1 variant library prepared in Experimental Example 1 was administered in the form of an aerosol into the trachea of 8-week-old male C57BL6 mice using a PenWu microaerosolizer (BioJane, Shanghai, China) (1.25" length of intratracheal portion, 700 µm outer diameter, 430 µm inner diameter), and necropsy was performed 2 weeks later. Smooth muscle cells were isolated from the lung tissue obtained from the necropsy by flow cytometry. During the flow cytometry, the smooth muscle cells were labeled and isolated using APC-conjugated α-SMA antibodies (Abcam, Cambridge, UK). The genes of the AAV1 variants were recovered from the isolated lung smooth muscle cells using Qiagen 51306.

The recovered capsid DNA was amplified and inserted into a plasmid to perform transformation into *E. coli*, and a library was screened through cloning. An AAV variant library was prepared from the library, and the process of aerosol administration into the mouse airway and screening was repeated a total of three times. The information of the AAV1-based variants screened through Sanger sequencing of the capsid DNA recovered from multiple single colonies obtained after transformation into *E. coli* during the library screening process is shown in Table 1. Among the screened variants, a variant in which the serine at the 430th position of the wild-type AAV1 capsid amino acid sequence is replaced with cysteine (S430C) and the isoleucine at the 647th position is replaced with valine (I647V) was named AAV1.p2CV. In addition, a variant in which serine at the 516th position is replaced with threonine (S516T), asparagine at the 583rd position is replaced with aspartic acid (N583D), and alanine at the 660th position is replaced with valine (A660V) was named AAVp5. In addition, a variant in which lysine (K) at the 310th position is replaced with arginine (R) (K310R) was named AAVp3.1. In addition, a variant in which tryptophan (W) at the 23rd position is replaced with arginine (R) (W23R) and threonine (T) at the 415th position is replaced with isoleucine (I) (T415I) was named AAVp3.2. In addition, a variant in which the asparagine at the 14th position is replaced with threonine (N14T), the glutamic acid at the 21st position is replaced with glutamine (E21Q), the aspartic acid at the 24th position is replaced with lysine (D24K), the lysine at the 31st position is replaced with threonine (K31T), the proline at the 32nd position is replaced with threonine (P32T), the asparagine at the 35th position is replaced with arginine (N35R), the glycine 36th position is replaced with glutamic acid (Q36E), the glutamine at the 37th position is replaced with arginine (Q37R), the lysine at the 38th position is replaced with histidine (K38H), the glutamine at the 39th position is replaced with lysine (Q39K), and the glycine at the 42nd position is replaced with serine (G42S) was named AAVp3.44. In addition, a variant in which the glutamine at the 688th position is replaced with arginine (Q688R) was named AAVp3.55. In addition, a variant in which the threonine at the 492nd position is replaced with alanine (T492A) was named AAVp3.55. In addition, a variant in which the lysine at the 137th position is replaced with glutamate (K137E) was named AAVp3.97.

During the above process, the recovered DNA was amplified by PCR, and the capsid genes of the library selected from lung smooth muscle cells were read using the Pacbio-SMRT (Pacific Bioscience) next-generation sequencing (NGS) equipment. Mutation information was collected from the NGS results, and the variants containing point mutations shown in Table 1 are shown in Table 2.

During the above process, there were variants found in duplicate in both the Sanger sequencing and NGS results, which were p2CV, AAVp5, AAVp3.97, AAVp3.56, AAVp3.55, AAVp3.2, and AAVp3.1. Additionally, variants containing point mutations shown in Table 1 were indicated by adding "partial_" to the variant characteristics shown in Table 2, and a wide variety of combinations of mutations different from those in Table 1 were found in the NGS results for the lung smooth muscle cells. Through this, it can be seen that variants consisting of combinations of point mutations present in the variant with enhanced lung smooth muscle cell tropism can enhance the tropism for lung smooth muscle cells.

**[Table 1]**

| Point mutations | SEQ ID NO | Variant name |
|---|---|---|
| S430C, I647V | 2 | AAV1.p2CV |
| S516T, N583D, A660V | 3 | AAVp5 |
| K310R | 4 | AAVp3.1 |
| W23R, T415I | 5 | AAVp3.2 |
| N14T, E21Q, D24K, K31T, P32T, N35R, Q36E, Q37R, K38H, Q39K, G42S | 6 | AAVp3.44 |
| Q688R | 7 | AAVp3.55 |
| T492A | 8 | AAVp3.56 |
| K137E | 9 | AAVp3.97 |

**[Table 2]**

| Point mutations | Variant characteristics |
|---|---|
| S430C, I647V | AAV1.p2CV |
| K310R | AAVp3.1 |
| W23R, T415I | AAVp3.2 |
| Q688R | AAVp3.55 |
| T492A | AAVp3.56 |
| K137E | AAVp3.97 |
| S516T, N583D, A660V | AAVp5 |
| I647V | partial_AAV.p2CV |
| Y414C, I647V | partial_AAV.p2CV |
| T242S, D556G, I647V | partial_AAV.p2CV |
| S430C | partial_AAV.p2CV |
| L53I, I647V | partial_AAV.p2CV |
| K123R, I647V | partial_AAV.p2CV |
| D9G, D556G, I647V | partial_AAV.p2CV |
| K31Q, D154G, K310R, V582G, M599V | partial_AAVp3.1 |
| K310R, S663T | partial_AAVp3.1 |
| K310R, A598V | partial_AAVp3.1 |
| W23R | partial_AAVp3.2 |
| W23R, F173L | partial_AAVp3.2 |
| N14T, E21Q, D24K, A29P, K31P, A34P, N35A, Q36E, Q37R, K38H, Q39K, G42S, A67E, Q81R, K84D, A85S, R92K, Q105K | partial_AAVp3.44 |
| N14T, E21Q, D24K, A29P, K31P, A34P, N35A, Q36E, Q37R, K38H, Q39K, G42S | partial_AAVp3.44 |
| N14T, E21Q, D24K, A29P, K31P, A34P, N35A, Q36E, Q37R, K38H, Q39K, G42S, A67E, Q81R, K84D, A85S, R92K, Q105K, G135P, A136V, Q148H, Q151V, I159T, T162A, K168R, S179A, E180D, E190Q, T194A, A196S, A197G, V198L, P200T, T201N, S205T, G207S | partial_AAVp3.44 |
| N14T | partial_AAVp3.44 |
| Q37R | partial_AAVp3.44 |
| N14T, E21Q, D24K, A29P, K31P, A34P, N35A, Q36E, Q37R, K38H, Q39K, G42S, A67E, Q81R, K84D, A85S, R92K, Q105K, G135P, A136V, Q148H, Q151V, I159T, T162A, K168R, S179A, E180D, E190Q, T194A, A196S, A197G, V198L, P200T, T201N, S205T, G207S, A224S | partial_AAVp3.44 |
| N14T, E21Q, D24K, A29P, K31P, A34P, N35A, Q36E, Q37R, K38H, Q39K, G42S, A67E | partial_AAVp3.44 |
| N14T, E21Q, D24K, A29P, K31P, A34P, N35A, Q36E, Q37R, K38H, Q39K, G42S, A67E, Q81R, K84D, A85S, R92K, Q105K, G135P, A136V, Q148H, Q151V, I159T, T162A, K168R | partial_AAVp3.44 |
| N14T, E21Q, D24K, A29P, K31P, A34P, N35A, Q36E, Q37R, | partial_AAVp3.44 |
| K38H, Q39K, G42S, A67E, Q81R, K84D, A85S, R92K | |
| K31T, M203K | partial_AAVp3.44 |
| N14T, E21Q, D24K, A29P, K31P, A34P, N35A, Q36E, Q37R, K38H, Q39K, G42S, A67E, Q81R, K84D, A85S, R92K, Q105K, G135P, A136V | partial_AAVp3.44 |
| N14T, E21Q, D24K, A29P, K31P, A34P, N35A, Q36E, Q37R, K38H, Q39K, G42S, A67E, Q81R, K84D, A85S, R92K, Q105K, G135P, A136V, Q148H, Q151V, I159T, T162A, K168R, S179A, E180D, E190Q, T194A, A196S, A197G, V198L, P200T, T201N, S205T, G207S, A224S, L235M | partial_AAVp3.44 |
| N14T, E21Q, D24K, A29P, K31P, A34P, N35A, Q36E, Q37R, K38H, Q39K, G42S, A67E, Q81R, K84D, A85S, R92K, Q105K, G135P, A136V, Q148H, Q151V, I159T, T162A, K168R, S179A, E180D, E190Q, T194A, A196S, A197G, V198L, P200T, T201N | partial_AAVp3.44 |
| N14T, E21Q, D24K, A29P, K31P, A34P, N35A, Q36E, Q37R, K38H, Q39K, G42S, A67E, Q81R, K84D, A85S, R92K, Q105K, G135P, A136V, Q148H, Q151V, I159T, T162A, K168R, S179A, E180D | partial_AAVp3.44 |
| N14T, E21Q, D24K, A29P, K31P, A34P, N35A, Q36E, Q37R, K38H, Q39K, G42S, A67E, G135P, A136V, H272T | partial_AAVp3.44 |
| N14T, E21Q | partial_AAVp3.44 |
| K31T | partial_AAVp3.44 |
| P32T, S385G | partial_AAVp3.44 |
| N14T, T138A, S205T | partial_AAVp3.44 |
| N14T, L129F, N383D, V558I | partial_AAVp3.44 |
| N14T, L129F, N383D | partial_AAVp3.44 |
| N14T, L129F | partial_AAVp3.44 |
| N14T, D24T, F56G | partial_AAVp3.44 |
| K31T, M203K, S663T | partial_AAVp3.44 |
| G42S | partial_AAVp3.44 |
| E21Q, D24K, A29P, K31P, A34P, N35A, Q36E, Q37R, K38H, Q39K, G42S, Y273H | partial_AAVp3.44 |
| D24K, A29P, K31P, A34P, N35A, Q36E, Q37R, K38H, Q39K, G42S, A67E, Q81R, K84D, A85S, R92K, Q105K, G135P, A136V, Q148H, Q151V, I159T, T162A, K168R, S179A, E180D, E190Q, T194A, A196S, A197G, V198L, P200T, T201N | partial_AAVp3.44 |
| D24A, K31T, K84Q, T589A, I683V | partial_AAVp3.44 |
| G42S, T492A | partial_AAVp3.44, partial_AAVp3.55 |
| G42S, A67E, Q81R, K84D, A85S, R92K, Q105K, G135P, A136V, Q148H, Q151V, I159T, T162A, K168R, S179A, E180D, E190Q, T194A, A196S, A197G, V198L, P200T, T201N, S205T, G207S, A224S, L235M, T492A | partial_AAVp3.44, partial_AAVp3.56 |
| N14T, E21Q, Q688R | partial_AAVp3.44, partial_AAVp3.56 |
| K31Q, M599V, Q688R | partial_AAVp3.55 |
| I159V, Q688R | partial_AAVp3.55 |
| D24A, Q688R | partial_AAVp3.55 |
| T326A, Q688R | partial_AAVp3.55 |
| Q688R, N718D | partial_AAVp3.55 |
| Q386H, Q688R | partial_AAVp3.55 |
| P571H, Q688R | partial_AAVp3.55 |
| P32S, P191L, P571H, Q688R | partial_AAVp3.55 |
| N458S, Q688R | partial_AAVp3.55 |
| M211V, K641M, Q688R | partial_AAVp3.55 |
| K641M, Q688R | partial_AAVp3.55 |
| G189R, Q688R | partial_AAVp3.55 |
| G163S, M211V, K641M, Q688R | partial_AAVp3.55 |
| A196V, Q688R | partial_AAVp3.55 |
| T176A, T492A | partial_AAVp3.56 |
| S205A, T492A | partial_AAVp3.56 |
| N172K, T492A | partial_AAVp3.56 |
| D24A, T492A | partial_AAVp3.56 |
| T492A, V654I | partial_AAVp3.56 |
| T492A, T589A | partial_AAVp3.56 |
| T492A, S588N | partial_AAVp3.56 |
| T492A, S547G | partial_AAVp3.56 |
| T492A, R694H | partial_AAVp3.56 |
| T492A, N717T | partial_AAVp3.56 |
| T492A, N496S | partial_AAVp3.56 |
| T492A, K533R | partial_AAVp3.56 |
| T492A, I727V | partial_AAVp3.56 |
| T492A, G544V | partial_AAVp3.56 |
| T492A, F638L, G639W, L640T, K641Q, N642E, P644A, P645S, Q646S, I647D, L648P, I649H, K650Q | partial_AAVp3.56 |
| T492A, A709T | partial_AAVp3.56 |
| S385G, T492A, N710S | partial_AAVp3.56 |
| S385G, T492A | partial_AAVp3.56 |
| S109T, T492A | partial_AAVp3.56 |
| Q164R, T492A | partial_AAVp3.56 |
| P420S, T492A | partial_AAVp3.56 |
| N35Y, T492A | partial_AAVp3.56 |
| N302K, T492A | partial_AAVp3.56 |
| N214S, T492A | partial_AAVp3.56 |
| L256I, T492A | partial_AAVp3.56 |
| L129F, T492A | partial_AAVp3.56 |
| K77E, T492A | partial_AAVp3.56 |
| K38R, E418D, T492A, N657T | partial_AAVp3.56 |
| K33R, V125I, V132A, T492A, S703Y | partial_AAVp3.56 |
| K169R, T492A | partial_AAVp3.56 |
| K123R, T492A | partial_AAVp3.56 |
| G174C, T492A, T589A | partial_AAVp3.56 |
| G135A, T492A | partial_AAVp3.56 |
| G112C, T411A, T492A | partial_AAVp3.56 |
| F399S, T492A | partial_AAVp3.56 |
| D24A, L129F, T492A | partial_AAVp3.56 |
| K137E, K169R, N451S | partial_AAVp3.97 |
| N14T, E21Q, D24K, A29P, K31P, A34P, N35A, Q36E, Q37R, K38H, Q39K, G42S, A67E, Q81R, K84D, A85S, R92K, Q105K, G135P, A136V, Q148H, Q151V, I159T, T162A, K168R, S179A, E180D, E190Q, T194A, A196S, A197G, V198L, P200T, T201N, S205T, G207S, A224S, L235M, P250S | partial_AAVp3.44 |
| N583D | partial_AAVp5 |
| A660V | partial_AAVp5 |
| S516T, N583D | partial_AAVp5 |
| A660V, D712G | partial_AAVp5 |
| S516T | partial_AAVp5 |
| N583D, A660V | partial_AAVp5 |
| S499G, E575G, M599I, A660V | partial_AAVp5 |
| S181L, A660V | partial_AAVp5 |
| P475L, A660V, D712G | partial_AAVp5 |
| K77E, S516T, N583D, A660V | partial_AAVp5 |
| G594R, A660V, D712G | partial_AAVp5 |
| G539D, A660V, D712G | partial_AAVp5 |
| G356D, S516T, N583D | partial_AAVp5 |
| D24A, N583D, S680N | partial_AAVp5 |
| A71V, A660V, D712G | partial_AAVp5 |
| A3V, D24A, K84Q, N583D | partial_AAVp5 |

### Experimental Example 3: Preparation of adeno-associated virus variants containing AAV1-based point mutations

The capsid gene recovered from the process of Experimental Example 2 and identified by Sanger sequencing was subcloned into pXX2 (UC Berkeley, David Schaffer Lab) containing HindIII and NotI to complete the construction of lung smooth muscle cell-specific plasmid variants for carrying the CMV-GFP and CMV-LacZ genes.

17 µg of the constructed adeno-associated virus variant plasmid containing AAV1-based point mutations, 17 µg of ITR-flanked reporter gene (pCMV-GFP or pCMV-LacZ or pCMV-FGF12-IRES-GFP), and 17 µg of pHelper were transfected into HEK293T cells as a calcium-phosphate complex, and after about 48 hours, only the cell pellets were collected and the AAV inside the cells was extracted through freezing and thawing. Subsequently, cell debris was removed by centrifugation, and the AAV solution was obtained by removing nucleic acids from virus-producing cells through incubation with 10 U/mL of Benzonase at 37 °C for 30 minutes. The AAV solution was purified by iodixanol density gradient ultracentrifugation (OptiPrep, Alere Technologies AS, Oslo, Norway) with a Vti65.2 rotor (Beckman Coulter; 42,000 rpm). The purified AAV vector was buffer-exchanged with a 1x PBS solution containing 0.01% (v/v) Tween 20 (Sigma-Aldrich) using an Amicon tube (Merck Millipore, Billerica, MA; molecular weight cutoff: 100 kDa).

### Experimental Example 4: Histological analysis for observing lung tissue specificity of adeno-associated virus variants containing AAV1-based point mutations

The adeno-associated virus variants AAVp5, AAVp3.1, AAVp3.2, AAVp2CV, AAVp3.44, AAVp3.55, AAVp3.56, and AAVp3.97 carrying CMV-LacZ as produced in Experimental Example 3 were administered in the form of an aerosol into the trachea of 8-week-old male C57BL6 mice using a PenWu microaerosolizer (BioJane, Shanghai, China) (1.25" length of intratracheal portion, 700 µm outer diameter, 430 µm inner diameter). Lung tissues were excised 3 weeks later, and LacZ expression was confirmed through X-gal staining.

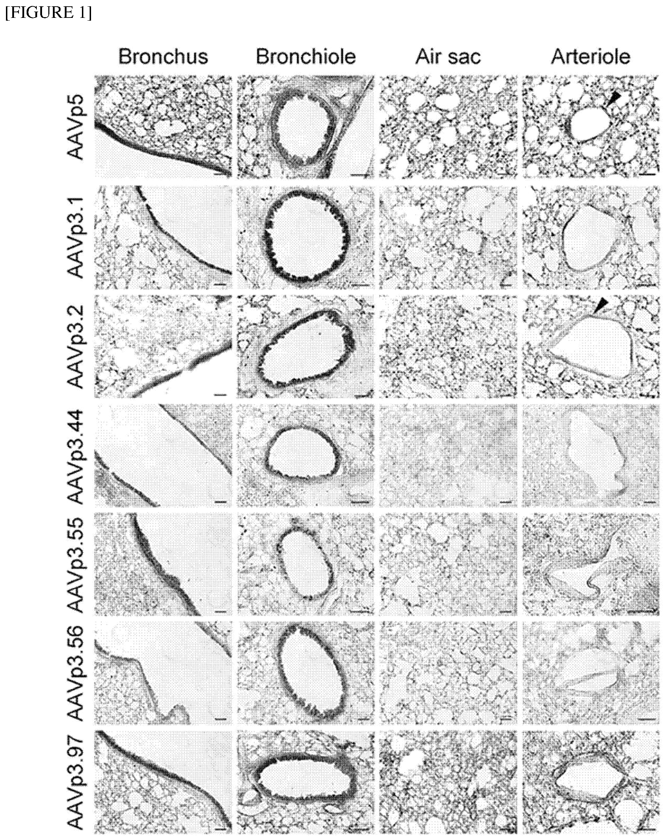

As a result, high LacZ gene expression was confirmed throughout the lung tissue for AAVp5, and the remaining variants were also confirmed to be capable of effectively delivering the LacZ gene to the lung trachea or bronchi (FIG. 1).

### Experimental Example 5: Confirmation of ability of delivery to pulmonary artery smooth muscle cells for individual point mutations of AAV1.p2CV

AAV1.p2CV is known to have high diffusivity and to effectively deliver packaged genes to pulmonary artery smooth muscle cells via airway aerosol administration (see Korean Patent Application No. 10-2022-0120442). Accordingly, the effect of point mutations S430C and I647V for the wild-type AAV1, which are possessed by AAV1.p2CV, on the tropism for pulmonary artery smooth muscle cells was investigated. A variant in which the serine at the 430th position of the AAV1 amino acid sequence is replaced with cysteine (S430C) was named AAV1.S430C, and a variant in which the isoleucine at the 647th position is replaced with valine (I647V) was named AAV1.I647V. These variants were prepared in the same manner as in Experimental Example 3.

Wild-type AAV1 and the variants AAV1.S430C, AAV1.I647V, and AAV1.p2CV were administered to male C57BL/6 mice via the airway in the same manner as in Experimental Example 2. On days 1 and 2, the mice were necropsied, and the process of AAV migration to pulmonary artery smooth muscle cells was imaged by immunohistochemistry. For the immunohistochemistry, AAV in lung tissue sections obtained from the necropsy was stained with Alexa594 (Thermo Fisher Scientific), the lung smooth muscle cells were stained with Alexa488-conjugated anti-α-SMA primary antibodies (Abcam; 1:100), and cell nuclei were stained with DAPI.

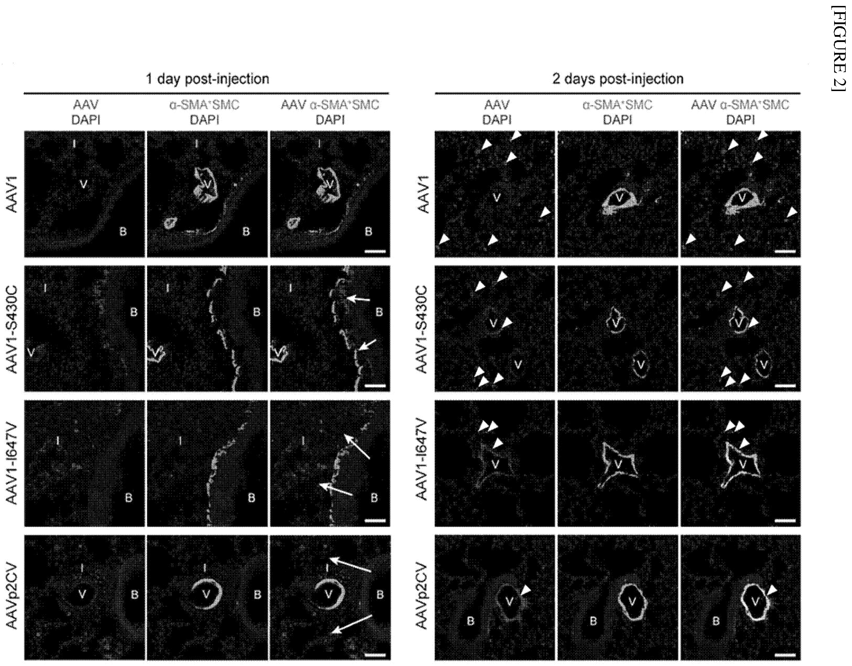

On day 1, the wild-type AAV1 showed very low infectivity around the pulmonary artery (V) in the image, while AAV1.S430C had reached the bronchial (B) smooth muscle cells, AAV1.I647V had moved beyond the bronchial smooth muscle cells, and AAV1.p2CV had reached the pulmonary artery periphery. On day 2, the wild-type AAV1 was observed around the pulmonary artery but its signal did not overlap with those of the pulmonary artery smooth muscle cells, while AAV1.S430C had its signal overlap with the pulmonary artery smooth muscle cells but was widely spread around the pulmonary artery periphery, AAV1.I647V had a large signal overlap with the pulmonary artery smooth muscle cells and was expressed in small amounts near the pulmonary artery, and AAV1.p2CV had a completely overlapping signal with the pulmonary artery smooth muscle cells (FIG. 2). Accordingly, it was confirmed that when the S430C mutation and the I647V mutation are combined, a synergistic effect occurs, further enhancing tropism for pulmonary artery smooth muscle cells.

### Experimental Example 6: Confirmation of diffusivity for individual point mutations and combinations of AAV1.p2CV

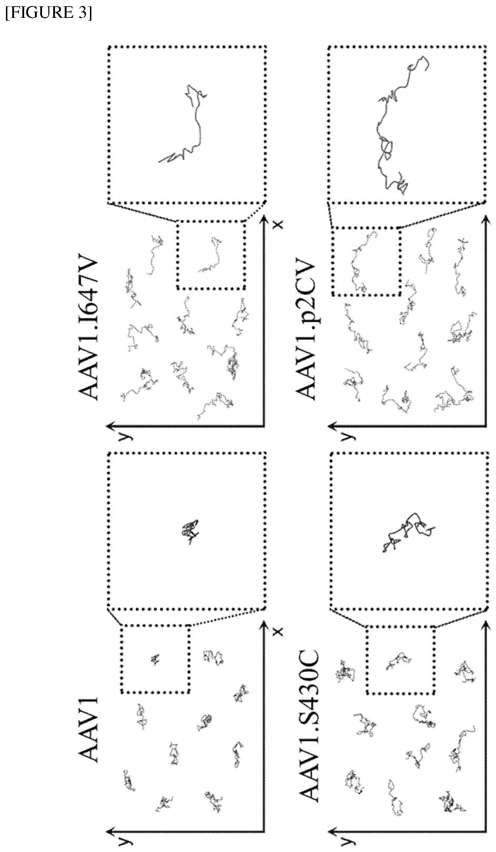

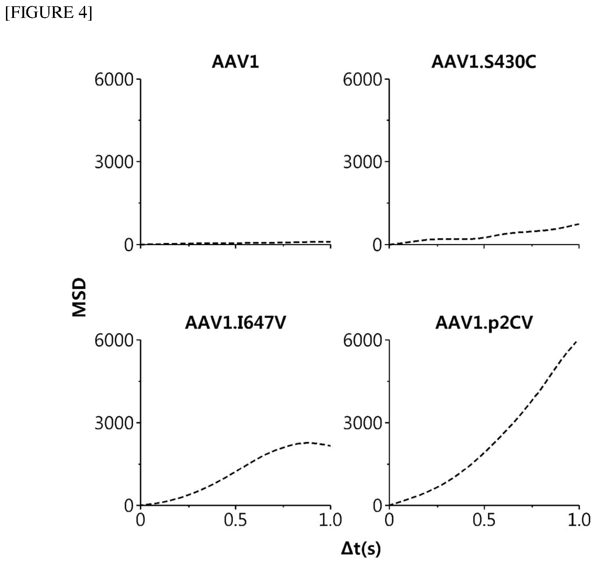

The movement trajectories of the wild-type AAV1 and the variants AAV1.S430C, AAV1.I647V, and AAV1.p2CV used in Experimental Example 5 were monitored for 1 second in a pH 7.4 PBS using the NanoSights (Malvern Panalytical) equipment. As shown in FIG. 3, the variants AAV1.S430C, AAV1.I647V, and AAV1.p2CV traveled a greater distance in the same amount of time as compared to the wild-type AAV1. Regarding the change in mean squared distance (MSD) depending on the length of observation time, the wild-type AAV1 showed maintenance at 0 as the observation time increased, while AAV1.S430C showed a slight increase and AAV1.I647V showed a rapid increase followed by maintenance. AAV1.p2CV, which contained both mutations, showed a significantly steep increase (FIG. 4). This shows that when the point mutations are combined, diffusivity can be improved significantly due to synergistic effect.

### Experimental Example 7: Confirmation of change in diffusivity depending on change of individual point mutation in AAV1.p2CV

The change in diffusivity was observed while replacing the 430th and 647th amino acids, which are the two point mutation sites of AAV1.p2CV, with different types. The variant in which the serine at the 430th position of the AAV1 amino acid sequence was replaced with arginine (S430R) was named AAV1.S430R, the variant in which the serine at the 430th position was replaced with glutamic acid (S430E) was named AAV1.S430E, the variant in which the isoleucine at the 647th position was replaced with alanine (I647A) was named AAV1.I647A, and the variant in which the isoleucine at the 647th position was replaced with tyrosine (I647Y) was named AAV1.I647Y.

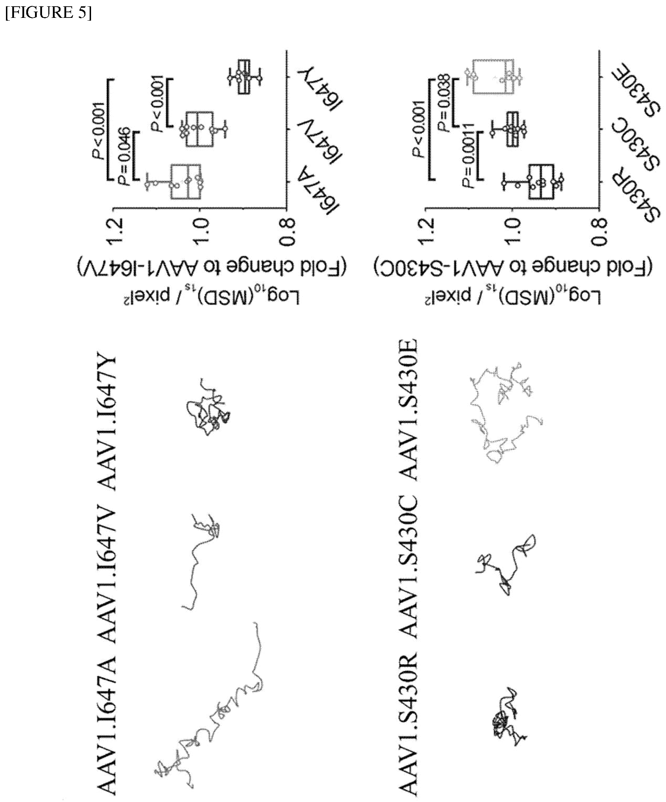

The trajectories of the variants AAV1.I647A, AAV1.I647V, AAV1.I647Y, AAV1.S430R, AAV1.S430C, and AAV1.S430E were monitored for 1 second in the same manner as in Experimental Example 6 (FIG. 5). AAV1.I647A traveled further than AAV1.I647V, and AAV1.I647Y traveled less. Additionally, AAV1.S430R traveled less than AAV1.S430C, and AAV1.S430E traveled further. The MSD values calculated from the experiment are shown on the right side of FIG. 5.

Tyrosine has a molecular weight of 181.191 Da, valine has 117.148 Da, and alanine has 89.094 Da. It was found that the diffusivity of the variant with the 647th amino acid substituted is affected by the molecular weight. Also, whereas the VP1 protein has a negative charge of -15.0 at pH 7.4, when the serine at the 430th position is replaced with arginine, the negative charge becomes -13.6, and when it is substituted with glutamic acid, it becomes -15.6. Thus, it can be seen that when the 430th amino acid of wild-type AAV1 is replaced, the diffusivity of the variant is proportional to the magnitude of the negative charge.

### Experimental Example 8: Confirmation of delivery capability to human pulmonary artery smooth muscle cells (hPASMCs) for individual point mutations in AAV1.p2CV

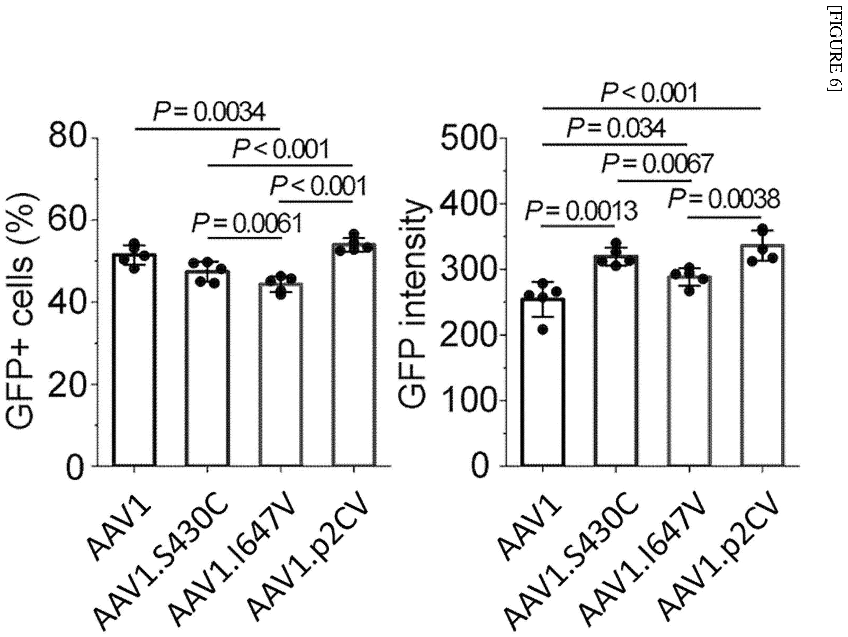

CMV-GFP was loaded onto the wild-type AAV1 and the variants AAV1.S430C, AAV1.I647V, and AAV1.p2CV produced in Experimental Example 3 and delivered to human pulmonary artery smooth muscle cells (hPASMCs) *in vitro*. The ratio of GFP-expressing cells and the average brightness (intensity) of the GFP signal were measured and are shown in FIG. 6. As a result of comparing the GFP expression rates of the four AAV-based viral vectors, the values for AAV1.S430C and AAV1.I647V were slightly reduced as compared to the wild-type AAV1, but AAV1.p2CV, which possesses both point mutations simultaneously, showed an expression rate equivalent to that of AAV1. In terms of intensity, AAV1.I647V showed a slightly lower value as compared to the wild-type AAV1, but AAV1.S430C and AAV1.p2CV showed increased values. Thus, it can be seen that the combination of S430C and I647V mutations has a synergistic effect on gene delivery capability and expression level.

### Experimental Example 9: Preparation of mouse model of pulmonary arterial hypertension

Generation of a mouse model of pulmonary arterial hypertension and animal experiments were performed according to the protocol approved by the IACUC of KAIST (No. KA2024-012-v1), in compliance with the guidelines for the care and use of experimental animals issued by the National Institutes of Health.

To minimize the potential effects of hormonal and sex differences on functional and histological results, adult male C57BL/6 mice at 10 weeks of age were used. The mice were maintained on a 12-hour light-dark cycle and allowed to consume food without restriction. The mice were housed in hypoxic cages with an oxygen concentration of 8.5% of atmospheric pressure for 3 weeks while administering Sugen 5416 (Cat No.: S8442, Sigma-Aldrich) at a dose of 20 mg/kg every week.

### Experimental Example 10: Preparation of 7-mer peptide-inserted adeno-associated virus libraries based on AAV1 and AAV1.p2CV

The AAV1 capsid gene and the AAV1.p2CV capsid gene obtained in Experimental Example 2 were subcloned into the pSub2 (UC Berkeley, David Schaffer Lab) plasmid to obtain the plasmids pSub2-AAV1 and pSub2-AAV1.p2CV for 7-mer peptide insertion. To prevent the generation of stop codons, synthesized 21-mer DNA duplexes consisting of 7 NNK codons with guanine or thymine following two random DNA bases (NN) were inserted between the amino acids at positions 588 and 589 of the capsid amino acid sequence of the wild-type AAV1 in the plasmid (between the nucleic acids at positions 1764 and 1765 of the DNA), thereby preparing AAV1 and AAV1.p2CV-based 7-mer peptide-inserted plasmid libraries.

The prepared AAV1 and AAV1.p2CV-based 7-mer peptide-inserted plasmids and pHelper and pBluescript plasmids were complexed using PEIpro (Polyplus, Cat No.: 101000033) and transfected into HEK293T cells. After about 72 hours, only the cell pellets were collected and the AAV inside the cells was extracted by freezing and thawing. Subsequently, cell debris was removed by centrifugation, and the AAV solution was obtained by removing nucleic acids from the virus-producing cells by incubating with 10 U/mL of Benzonase at 37 °C for 30 minutes. The AAV solution was purified by iodixanol density gradient ultracentrifugation (OptiPrep, Alere Technologies AS, Oslo, Norway) with a Vti65.2 rotor (Beckman Coulter; 42,000 rpm). The purified AAV vector was buffer-exchanged with a 1x PBS solution containing 0.01% (v/v) Tween 20 (Sigma-Aldrich) using an Amicon tube (Merck Millipore, Billerica, MA; molecular weight cutoff: 100 kDa).

### Experimental Example 11: Screening of lung smooth muscle cell-targeting variants from AAV1 and AAV1.p2CV-based 7-mer peptide-inserted adeno-associated virus libraries in mouse model of pulmonary arterial hypertension

The AAV1 and AAV1.p2CV-based 7-mer peptide-inserted variant libraries were administered, respectively, in the form of an aerosol into the airway of 10-week-old male C57BL6 mice using a PenWu microaerosolizer (BioJane, Shanghai, China) (1.25" length of intratracheal portion, 700 µm outer diameter, 430 µm inner diameter). After approximately 1 day of recovery, pulmonary arterial hypertension was induced according to Experimental Example 9. Immediately after the completion of a 3-week pulmonary arterial hypertension induction process, perfusion was performed, followed by the extraction of major organs (lungs, liver, kidneys, heart). Pulmonary smooth muscle (PSMC) cells labeled with APC-conjugated α-SMA antibodies (Abcam, Cambridge, UK) were isolated by flow cytometry of the extracted lung tissues. The lung cells remaining after the isolation were designated as "negative." 7-mer peptide-inserted neighboring genes of the AAV1 and AAV1.p2CV-based variants were recovered from PSMC, negative, liver, kidney, and heart cells using Qiagen 51306. Subsequently, DNA amplification was performed via PCR, and the inserted genes were read using the Illumina MiSeq (Illumina, Inc.) next-generation sequencing (NGS) instrument. In addition, this process allowed the mutation information of the 7-mer peptides inserted into the wild-type AAV1 to be collected for score calculation. The inserted DNAs read via NGS were converted into amino acid sequences to represent the inserted 7-mer peptides, and the number of NGS copies for each peptide was obtained for each tissue. The score was defined as the ratio of each 7-mer peptide sequence to the total NGS copies for each tissue, divided by the ratio of the corresponding 7-mer peptide sequence to the total NGS copies in the recombinant variant library.

Cell and tissue-specific specificity scores for PSMC, negative, liver, kidney, and heart were calculated for the 7-mer peptide-inserted variants screened by NGS using the method described above. The variants with higher lung smooth muscle cell scores as compared to the wild-type AAV1 were selected, and their insertion sequences are shown in Table 3 below. Additionally, the specificity scores for PSMC, negative, liver, kidney, or heart cells of the AAV1 capsid variants containing each insertion sequence are also shown. The NGS copies ratio of the corresponding inserted peptide in the variant library was defined as VG_freq, and the value was presented.

**[Table 3]**

| Inserted sequence | SEQ ID NO | VG_freq | PSMC | Negative | Liver | Kidney | Heart |
|---|---|---|---|---|---|---|---|
| GRPQNRA | 35 | 5.58422E-07 | 118.1851 | 0 | 0 | 23.6878 2 | 0 |
| IILSLRA | 36 | 5.47301E-07 | 175.049 4 | 0 | 0 | 44.3101 2 | 0 |
| HLSRCGP | 37 | 5.58422E-07 | 139.049 4 | 0 | 0 | 43.4276 8 | 0 |
| ARRLRLL | 38 | 5.58422E-07 | 172.276 2 | 0 | 0 | 55.2715 9 | 0 |
| SSPSLLS | 39 | 5.58422E-07 | 193.059 2 | 0 | 0 | 78.9594 1 | 0 |
| RPLSRHR | 40 | 5.58422E-07 | 79.7608 6 | 0 | 0 | 47.3756 5 | 0 |
| STSKGSV | 41 | 5.47301E-07 | 279.280 6 | 42.9131 4 | 18.5922 3 | 80.5638 6 | 0 |
| SGPVCVC | 42 | 1.11684E-06 | 109.436 4 | 17.3719 9 | 0 | 69.0894 9 | 0 |
| LGRASGC | 43 | 5.58422E-07 | 131.560 9 | 38.2764 5 | 0 | 55.2715 9 | 0 |
| PTLLTPT | 44 | 5.58422E-07 | 61.7276 5 | 0 | 0 | 94.7513 | 0 |
| ILSVKLP | 45 | 5.58422E-07 | 68.6907 | 0 | 0 | 106.595 2 | 0 |
| RSALLRD | 46 | 5.58422E-07 | 451.757 1 | 230.407 5 | 0 | 71.0634 7 | 0 |
| GRKRART | 47 | 5.58422E-07 | 160.459 7 | 67.6593 5 | 0 | 161.866 8 | 6.33466 8 |
| TRMRNGT | 48 | 5.47301E-07 | 235.693 7 | 126.926 2 | 0 | 56.3947 | 0 |
| AATARST | 49 | 5.58422E-07 | 379.972 6 | 222.423 4 | 0 | 146.074 9 | 6.33466 8 |
| ASLLSKL | 50 | 5.58422E-07 | 138.786 7 | 103.346 4 | 0 | 67.1155 | 0 |
| GSRAKTR | 51 | 5.47301E-07 | 131.111 5 | 98.8867 9 | 0 | 84.5920 5 | 0 |
| FVLLRAV | 52 | 5.58422E-07 | 136.232 8 | 106.060 6 | 0 | 0 | 0 |
| SPPLLPC | 53 | 5.47301E-07 | 288.131 5 | 242.136 2 | 0 | 8.05638 6 | 0 |
| SVMRRPR | 54 | 5.47301E-07 | 89.5689 | 72.2503 2 | 0 | 108.761 2 | 0 |
| SPMTLAT | 55 | 5.58422E-07 | 248.803 7 | 47.5444 | 0 | 841.743 5 | 0 |
| PLGPAKR | 56 | 5.58422E-07 | 122.221 2 | 135.881 4 | 0 | 7.89594 1 | 0 |
| TDSSTLD | 57 | 5.47301E-07 | 178.586 8 | 210.892 8 | 0 | 40.2819 3 | 0 |
| VLTQSVV | 58 | 5.47301E-07 | 81.2113 2 | 91.4236 4 | 0 | 128.902 2 | 0 |
| LGQSNVL | 59 | 5.58422E-07 | 158.704 4 | 195.209 9 | 21.2409 4 | 78.9594 1 | 12.6693 4 |
| HKLRCLP | 60 | 5.47301E-07 | 119.089 | 145.531 5 | 0 | 157.099 5 | 12.9267 8 |
| PKVLGGA | 61 | 5.47301E-07 | 81.2113 2 | 103.493 7 | 0 | 140.986 8 | 0 |
| FLLVVVS | 62 | 5.47301E-07 | 60.0741 3 | 83.9665 8 | 0 | 16.1127 7 | 0 |
| TKQTKKT | 63 | 5.47301E-07 | 159.093 9 | 268.673 5 | 56.8904 5 | 52.3665 1 | 6.46338 8 |
| APSRRTV | 64 | 5.58422E-07 | 98.8510 2 | 204.347 1 | 0 | 15.7918 8 | 12.6693 4 |
| AASECAG | 65 | 5.58422E-07 | 90.4972 7 | 162.748 2 | 0 | 356.387 8 | 621.884 4 |
| SITSTSP | 66 | 5.47301E-07 | 112.218 5 | 274.270 9 | 0 | 4.02819 3 | 0 |
| FPVARTT | 67 | 5.58422E-07 | 171.410 9 | 151.356 4 | 0 | 1368.71 7 | 12.6693 4 |
| GRARGSD | 68 | 5.47301E-07 | 68.7591 9 | 242.552 5 | 0 | 116.817 6 | 0 |
| ASSPALS | 69 | 5.53974E-06 | 44.8819 6 | 166.157 | 5.87785 2 | 134.480 3 | 62.4852 9 |
| LLPSLKG | 70 | 5.58422E-07 | 38.1615 | 204.779 | 0 | 138.179 | 6.33466 8 |
| SLRQSDD | 71 | 5.58422E-07 | 46.5589 1 | 0 | 0 | 973.178 6 | 14111.1 3 |
| PTPAKPP | 72 | 5.47301E-07 | 126.431 9 | 0 | 0 | 0 | 0 |
| SAQRRVV | 73 | 1.11684E-06 | 95.1641 | 0 | 0 | 11.8439 1 | 0 |
| FTMPSVG | 74 | 5.47301E-07 | 209.223 8 | 0 | 0 | 32.2255 4 | 0 |
| LTPVRSC | 75 | 1.66415E-06 | 29.4572 6 | 0 | 0 | 0 | 11.3025 1 |
| RPSAALH | 76 | 5.58E-07 | 1.97E+ 02 | 0.00E+ 00 | 0.00E+ 00 | 1.18E+ 02 | 0.00E+ 00 |
| FDSFAVR | 77 | 5.58422E-07 | 281.350 9 | 0 | 0 | 59.2195 6 | 0 |
| SAGTISC | 78 | 5.47E-07 | 1.96E+ 02 | 8.77E+ 01 | 0.00E+ 00 | 9.26E+ 01 | 0.00E+ 00 |
| LIGNLTA | 79 | 5.47301E-07 | 164.505 3 | 0 | 0 | 40.2819 3 | 6.46338 8 |
| ASGKTPM | 80 | 5.47E-07 | 1.90E+ 02 | 2.54E+ 02 | 2.79E+ 01 | 1.57E+ 02 | 4.08E+ 01 |
| TGAAFSV | 81 | 5.47E-07 | 1.90E+ 02 | 0.00E+ 00 | 0.00E+ 00 | 2.42E+ 01 | 0.00E+ 00 |
| EPAVRHR | 82 | 1.11684E-06 | 78.6705 3 | 5.74146 8 | 0 | 15.7918 8 | 3.16733 4 |
| RPRTTYR | 83 | 5.47301E-07 | 60.6888 8 | 0 | 0 | 20.1409 6 | 0 |
| PAPESTE | 84 | 5.47E-07 | 1.73E+ 02 | 0.00E+ 00 | 2.71E+ 00 | 4.03E+ 01 | 0.00E+ 00 |
| MDPKSVR | 85 | 1.10572E-06 | 140.494 2 | 9.23511 4 | 0 | 51.8398 2 | 15.9959 5 |
| WSVATSS | 86 | 1.65302E-06 | 81.4045 | 0 | 0 | 36.0098 3 | 0 |
| VVMNRPA | 87 | 5.47301E-07 | 134.249 | 5.85813 4 | 0 | 60.4228 9 | 0 |
| PKPATKK | 88 | 5.47E-07 | 1.57E+ 02 | 0.00E+ 00 | 0.00E+ 00 | 4.03E+ 00 | 0.00E+ 00 |
| ETSERKV | 89 | 5.47301E-07 | 86.7780 9 | 0 | 0 | 44.3101 2 | 0 |
| VFLHVAP | 90 | 5.58422E-07 | 520.987 8 | 63.1561 5 | 0 | 185.554 6 | 6.33466 8 |
| PDVNARS | 91 | 5.58422E-07 | 166.843 4 | 0 | 0 | 98.6992 7 | 63.1929 8 |
| NRPQLLN | 92 | 5.47301E-07 | 192.264 1 | 1.86578 8 | 0 | 120.845 8 | 0 |
| FPGHCGY | 93 | 5.47E-07 | 1.51E+ 02 | 0.00E+ 00 | 0.00E+ 00 | 5.64E+ 01 | 1.27E+ 02 |
| NKKQKKP | 94 | 5.47301E-07 | 45.2992 2 | 0 | 0 | 32.2255 4 | 0 |
| LMTVPPT | 95 | 5.47301E-07 | 485.669 | 78.1084 5 | 0 | 165.155 9 | 421.699 6 |
| GGGILRS | 96 | 5.47301E-07 | 109.129 9 | 0 | 0 | 96.6766 3 | 0 |
| GGAIAHN | 97 | 5.58422E-07 | 53.4261 | 0 | 0 | 51.3236 2 | 0 |
| PWSLVVL | 98 | 5.47301E-07 | 127.803 8 | 5.85813 4 | 0 | 124.874 | 6.46338 8 |
| TAQQSER | 99 | 5.47301E-07 | 477.696 5 | 123.142 | 0 | 68.4792 8 | 696.948 1 |
| HPKKDGL | 100 | 5.58E-07 | 1.23E+ 02 | 0.00E+ 00 | 0.00E+ 00 | 2.37E+ 01 | 0.00E+ 00 |
| HVANRLC | 101 | 5.58422E-07 | 294.874 8 | 84.1170 2 | 0 | 165.814 8 | 0 |
| LLTRAMC | 102 | 5.58422E-07 | 34.8905 1 | 0 | 0 | 39.4797 1 | 0 |
| PGYLCGL | 103 | 5.58422E-07 | 76.7483 4 | 0 | 0 | 90.8033 2 | 0 |
| LSLLWPP | 104 | 5.58E-07 | 1.17E+ 02 | 0.00E+ 00 | 0.00E+ 00 | 3.95E+ 00 | 1.88E+ 02 |
| FGAHRFL | 105 | 5.58422E-07 | 85.5856 5 | 0 | 0 | 106.595 2 | 0 |
| PRALLCT | 106 | 5.58422E-07 | 27.2582 1 | 0 | 0 | 31.5837 7 | 42.1798 9 |
| ADSRAMD | 107 | 5.58422E-07 | 365.354 2 | 129.832 8 | 0 | 185.554 6 | 69.5276 5 |
| FCRVVVG | 108 | 5.47301E-07 | 106.798 5 | 11.7162 7 | 0 | 140.986 8 | 0 |
| GTEVPRR | 109 | 5.58422E-07 | 37.0711 7 | 0 | 0 | 55.2715 9 | 0 |
| MPSVVHR | 110 | 5.58422E-07 | 451.786 2 | 148.204 3 | 38.2661 3 | 225.034 3 | 1149.05 6 |
| PTTVMTP | 111 | 5.47E-07 | 1.11E+ 02 | 0.00E+ 00 | 0.00E+ 00 | 8.06E+ 01 | 7.81E+ 02 |
| IRILLHR | 112 | 5.47301E-07 | 221.690 8 | 124.973 5 | 65.0176 6 | 278.449 8 | 0 |
| RHSTLAT | 113 | 5.58422E-07 | 139.666 4 | 93.7773 1 | 0 | 63.1675 3 | 0 |
| LSTKRQS | 114 | 5.47301E-07 | 56.7366 8 | 0 | 0 | 145.014 9 | 0 |
| TRHPLPL | 115 | 5.58422E-07 | 88.3166 1 | 66.9837 9 | 0 | 15.7918 8 | 0 |
| HPSTNQP | 116 | 5.47301E-07 | 259.270 7 | 205.034 7 | 0 | 104.733 | 0 |
| VSVCVPC | 117 | 5.47301E-07 | 264.901 2 | 212.699 9 | 0 | 52.3665 1 | 0 |
| SSRHIRR | 118 | 5.47E-07 | 9.30E+ 01 | 0.00E+ 00 | 4.09E+ 01 | 3.63E+ 01 | 0.00E+ 00 |
| RAGVRCD | 119 | 5.58422E-07 | 149.106 3 | 120.570 8 | 0 | 35.5317 4 | 0 |
| RKIHPSP | 120 | 5.47301E-07 | 137.190 9 | 111.304 5 | 0 | 24.1691 6 | 0 |
| VESRPRN | 121 | 5.58422E-07 | 299.121 4 | 243.055 5 | 42.4818 8 | 59.2195 6 | 0 |
| WPQTPAG | 122 | 5.58422E-07 | 115.124 6 | 97.2641 9 | 0 | 11.8439 1 | 0 |
| KRASTLP | 123 | 5.47301E-07 | 33.3745 2 | 0 | 35.2179 | 96.6766 3 | 0 |
| SGSSEHG | 124 | 5.58422E-07 | 90.3868 6 | 566.491 5 | 123.909 4 | 15.7918 8 | 12.6693 4 |
| DSGLGRR | 125 | 5.58422E-07 | 155.917 | 130.139 9 | 37.1716 5 | 114.491 1 | 0 |
| FLIVASV | 126 | 5.47301E-07 | 225.842 9 | 195.907 8 | 0 | 80.5638 6 | 6.46338 8 |
| QLALVSR | 127 | 5.47301E-07 | 102.348 5 | 82.0946 9 | 0 | 128.902 2 | 6.46338 8 |
| VIRVSMS | 128 | 5.58422E-07 | 404.237 1 | 380.242 4 | 0 | 288.201 9 | 0 |
| AKRWGTN | 129 | 5.47301E-07 | 130.165 | 141.799 9 | 0 | 88.6202 4 | 0 |
| RGGKGPK | 130 | 1.11E-06 | 8.20E+ 01 | 0.00E+ 00 | 0.00E+ 00 | 3.39E+ 01 | 0.00E+ 00 |
| SWGAGDS | 131 | 1.10572E-06 | 94.1408 1 | 112.118 1 | 15.6444 5 | 0 | 34.0988 2 |
| HVQWGT N | 132 | 5.47301E-07 | 165.417 8 | 208.893 6 | 0 | 108.761 2 | 12.9267 8 |
| RRHRTRT | 133 | 5.58422E-07 | 554.869 3 | 151.861 6 | 0 | 2468.80 7 | 63.1929 8 |
| SMGSGPV | 134 | 5.58422E-07 | 110.308 9 | 148.119 1 | 0 | 59.2195 6 | 23.1758 8 |
| FRLKVGF | 135 | 5.47E-07 | 6.79E+ 01 | 8.82E+ 01 | 0.00E+ 00 | 4.03E+ 01 | 0.00E+ 00 |
| SDLISLG | 136 | 1.66415E-06 | 87.5630 6 | 97.1842 1 | 12.4733 5 | 282.345 9 | 58.0156 1 |
| CGSAKLT | 137 | 5.47301E-07 | 134.136 4 | 199.176 5 | 0 | 36.2537 4 | 0 |
| QTTEPST | 138 | 1.10572E-06 | 84.9029 8 | 94.7205 | 0 | 222.604 6 | 583.428 1 |
| YGVSSSR | 139 | 5.47301E-07 | 298.524 6 | 483.291 8 | 0 | 100.704 8 | 12.9267 8 |
| LADPRSV | 140 | 5.58422E-07 | 291.7621 | 477.9841 | 0 | 110.5432 | 0 |
| PDFLGVP | 141 | 5.47301E-07 | 201.2513 | 342.7614 | 0 | 100.7048 | 0 |
| SLGAWRG | 142 | 5.47E-07 | 5.87E+ 01 | 0.00E+ 00 | 0.00E+ 00 | 1.05E+ 02 | 0.00E+ 00 |
| VYVQVPR | 143 | 5.58422E-07 | 127.8166 | 227.7449 | 0 | 35.53174 | 0 |
| LPVTKED | 144 | 5.58422E-07 | 72.95615 | 137.1473 | 0 | 55.27159 | 0 |
| SAFKPDL | 145 | 5.58422E-07 | 197.7166 | 317.0982 | 0 | 688.5118 | 42.17989 |
| LDQKRQP | 146 | 5.58422E-07 | 123.063 3 | 177.088 | 0 | 579.202 2 | 0 |
| TPTDGTR | 147 | 5.58422E-07 | 66.7394 | 133.490 1 | 0 | 43.4276 8 | 0 |
| QVVVPPS | 148 | 5.47301E-07 | 301.779 1 | 635.864 1 | 0 | 80.5638 6 | 6.46338 8 |
| PAVHHAS | 149 | 2.04E-05 | 5.55E+ 01 | 1.07E+ 02 | 8.55E+ 00 | 1.26E+ 02 | 7.20E+ 02 |
| HKGLNKY | 150 | 5.47E-07 | 4.89E+ 01 | 0.00E+ 00 | 0.00E+ 00 | 3.22E+ 01 | 0.00E+ 00 |
| **SGVSILG** | 151 | 9.97375E-06 | 48.6354 | 96.0094 1 | 2.97315 7 | 286.220 4 | 1414.07 5 |
| **SPNSAGV** | 152 | 1.11017E-05 | 36.373 | 83.2703 3 | 2.93303 8 | 91.8542 1 | 32.1436 9 |
| PSPTRTH | 153 | 5.47E-07 | 4.45E+ 01 | 0.00E+ 00 | 0.00E+ 00 | 0.00E+ 00 | 0.00E+ 00 |
| SIARRIV | 154 | 5.47301E-07 | 131.326 4 | 336.596 | 0 | 157.099 5 | 6.46338 8 |
| PPDLVPN | 155 | 5.58422E-07 | 141.144 5 | 369.690 6 | 0 | 75.0114 4 | 0 |
| SSKIVPS | 156 | 6.6677E-06 | 35.1278 1 | 97.1316 4 | 0 | 52.3177 | 15.3725 1 |
| TQLVRKT | 157 | 5.58422E-07 | 53.4261 | 158.847 3 | 0 | 27.6357 9 | 0 |
| EAVTLKR | 158 | 5.58422E-07 | 120.768 | 404.127 4 | 0 | 55.2715 9 | 0 |
| FSVGNGA | 159 | 1.10572E-06 | 29.8507 9 | 100.519 7 | 26.8182 2 | 5.98151 7 | 6.39838 1 |
| RTTPPWL | 160 | 5.58422E-07 | 34.8905 1 | 129.327 6 | 0 | 35.5317 4 | 0 |
| NPLVPVP | 161 | 5.47301E-07 | 182.017 7 | 731.773 4 | 0 | 116.817 6 | 0 |
| QSKETVR | 162 | 5.58422E-07 | 37.0711 7 | 167.302 9 | 0 | 82.9073 8 | 0 |
| PSLSLYL | 163 | 5.58422E-07 | 57.7874 1 | 265.151 5 | 0 | 63.1675 3 | 0 |
| VWVKKKP | 164 | 5.58422E-07 | 101.567 4 | 532.882 6 | 0 | 47.3756 5 | 320.444 2 |
| HSGHVCV | 165 | 5.47301E-07 | 47.8368 1 | 263.076 2 | 0 | 56.3947 | 0 |
| DTPTPTT | 166 | 5.47301E-07 | 25.5871 3 | 150.358 8 | 0 | 0 | 0 |
| ARTQWLP | 167 | 5.47301E-07 | 24.4746 5 | 210.892 8 | 0 | 68.4792 8 | 0 |
| LRGDLLN | 168 | 1.11684E-06 | 38.0729 2 | 244.327 4 | 0 | 3.94797 1 | 49.5191 |
| ERTTRKV | 169 | 5.58422E-07 | 142.837 3 | 988.695 3 | 0 | 122.387 1 | 12.6693 4 |
| PTPDRTA | 170 | 5.47301E-07 | 40.0494 2 | 395.739 3 | 0 | 40.2819 3 | 0 |
| ARPKPNV | 171 | 5.47301E-07 | 342.698 3 | 0 | 0 | 0 | 0 |
| RISSQST | 172 | 5.58E-07 | 4.12E+ 02 | 1.06E+ 02 | 0.00E+ 00 | 7.50E+ 01 | 0.00E+ 00 |
| SAVHHAS | 173 | 5.58E-07 | 3.20E+ 02 | 1.91E+ 00 | 0.00E+ 00 | 5.63E+ 02 | 1.03E+ 02 |
| FIVCARV | 174 | 5.58422E-07 | 23.9872 3 | 0 | 0 | 0 | 0 |
| SEARSSR | 175 | 1.10572E-06 | 121.3447 | 0 | 0 | 1.993839 | 0 |
| GRSVFSG | 176 | 5.58E-07 | 2.33E+ 02 | 1.86E+ 02 | 0.00E+ 00 | 4.74E+ 01 | 0.00E+ 00 |
| NDTPPPR | 177 | 5.58422E-07 | 169.516 1 | 0 | 0 | 19.7398 5 | 0 |
| FSCRKRA | 178 | 5.47301E-07 | 33.37452 | 0 | 0 | 4.028193 | 0 |
| AQRQLAR | 179 | 5.47301E-07 | 176.03 | 0 | 0 | 24.16916 | 0 |
| AWQPVGA | 180 | 5.47301E-07 | 336.719 3 | 0 | 0 | 48.3383 1 | 75.3539 1 |
| SRLENQS | 181 | 5.47301E-07 | 223.320 2 | 0 | 0 | 40.2819 3 | 12.9267 8 |
| YAVVPLY | 182 | 5.47E-07 | 2.10E+ 02 | 1.41E+ 02 | 1.90E+ 01 | 2.42E+ 01 | 4.30E+ 01 |
| CTKISTS | 183 | 5.58422E-07 | 40.2129 2 | 0 | 0 | 0 | 19.004 |
| PRRDSQV | 184 | 1.11684E-06 | 91.4219 8 | 0 | 0 | 17.7658 7 | 0 |
| DRISTDA | 185 | 5.47301E-07 | 152.516 8 | 0 | 0 | 36.2537 4 | 0 |
| LSKYRAW | 186 | 5.58E-07 | 1.92E+ 02 | 2.16E+ 02 | 0.00E+ 00 | 4.34E+ 01 | 0.00E+ 00 |
| SNPTELT | 187 | 5.58E-07 | 1.92E+ 02 | 0.00E+ 00 | 0.00E+ 00 | 4.34E+ 01 | 0.00E+ 00 |
| AFFPVPL | 188 | 5.58422E-07 | 42.5228 1 | 0 | 0 | 11.8439 1 | 0 |
| ITRFKEC | 189 | 5.47301E-07 | 259.666 3 | 0 | 0 | 84.5920 5 | 0 |
| QKKTKTQ | 190 | 5.58E-07 | 1.77E+ 02 | 1.13E+ 02 | 0.00E+ 00 | 0.00E+ 00 | 6.33E+ 00 |
| GPDAARK | 191 | 5.58E-07 | 1.72E+ 02 | 0.00E+ 00 | 0.00E+ 00 | 3.95E+ 00 | 0.00E+ 00 |
| KKKKKKK | 192 | 1.09E-06 | 1.69E+ 02 | 2.31E+ 02 | 0.00E+ 00 | 3.02E+ 01 | 3.23E+ 00 |
| TQQTVPE | 193 | 1.11E-06 | 1.59E+ 02 | 2.61E+ 01 | 0.00E+ 00 | 7.98E+ 01 | 3.20E+ 00 |
| KWTQVSP | 194 | 5.47301E-07 | 163.973 5 | 11.7162 7 | 0 | 56.3947 | 6.46338 8 |
| VCLPWTS | 195 | 5.47E-07 | 1.57E+ 02 | 1.37E+ 02 | 0.00E+ 00 | 4.43E+ 01 | 0.00E+ 00 |
| NPPAREG | 196 | 5.58422E-07 | 141.244 6 | 10.9717 9 | 0 | 59.2195 6 | 0 |
| EVSTPNN | 197 | 5.58422E-07 | 196.884 7 | 0 | 0 | 98.6992 7 | 16.8412 1 |
| LFGRAGY | 198 | 5.47E-07 | 1.57E+ 02 | 3.91E+ 00 | 0.00E+ 00 | 1.21E+ 02 | 0.00E+ 00 |
| MKLYRLS | 199 | 1.0946E-06 | 23.3621 6 | 0 | 0 | 12.0845 8 | 0 |
| TEQKNHQ | 200 | 5.58E-07 | 1.57E+ 02 | 3.02E+ 02 | 0.00E+ 00 | 1.97E+ 01 | 0.00E+ 00 |
| SKWTAW A | 201 | 5.58422E-07 | 52.3357 7 | 0 | 2.65511 8 | 27.6357 9 | 0 |
| GFQWLPT | 202 | 5.58422E-07 | 150.000 6 | 0 | 32.7995 4 | 82.9073 8 | 0 |
| TKDNAPD | 203 | 5.58422E-07 | 285.941 6 | 34.4488 1 | 0 | 114.491 1 | 6.33466 8 |
| SFMSTNL | 204 | 5.47301E-07 | 267.224 | 44.7789 2 | 0 | 48.3383 1 | 0 |
| QRKPAHE | 205 | 5.47E-07 | 1.56E+ 02 | 4.25E+ 02 | 0.00E+ 00 | 7.65E+ 01 | 0.00E+ 00 |
| PRYWPSL | 206 | 5.47301E-07 | 53.3992 3 | 0 | 0 | 40.2819 3 | 0 |
| EGTPNIN | 207 | 1.10572E-06 | 141.030 1 | 25.8583 2 | 0 | 13.9568 7 | 142.716 |
| QKPQNTK | 208 | 5.47E-07 | 1.42E+ 02 | 0.00E+ 00 | 0.00E+ 00 | 2.82E+ 01 | 0.00E+ 00 |
| RPVDRFR | 209 | 5.58422E-07 | 45.7938 | 0 | 0 | 39.4797 1 | 0 |
| SFVRGYC | 210 | 5.47301E-07 | 112.146 1 | 0 | 0 | 96.6766 3 | 6.46338 8 |
| TDVPMDE | 211 | 5.47E-07 | 1.37E+ 02 | 7.68E+ 01 | 0.00E+ 00 | 3.63E+ 01 | 0.00E+ 00 |
| KGMPEPR | 212 | 5.58422E-07 | 123.584 5 | 375.124 9 | 31.8614 1 | 764.346 5 | 0 |
| TRSLESM | 213 | 5.47E-07 | 1.19E+ 02 | 5.22E+ 01 | 0.00E+ 00 | 1.21E+ 01 | 0.00E+ 00 |
| SCLGADL | 214 | 5.58422E-07 | 347.243 9 | 87.7742 9 | 47.7921 2 | 339.525 5 | 0 |
| TCHSRSV | 215 | 5.47301E-07 | 94.5611 3 | 0 | 0 | 32.2255 4 | 320.492 2 |
| LGVRVMH | 216 | 5.58E-07 | 1.15E+ 02 | 1.04E+ 02 | 0.00E+ 00 | 3.95E+ 01 | 0.00E+ 00 |
| RPFSHPT | 217 | 5.58E-07 | 1.11E+ 02 | 1.32E+ 02 | 0.00E+ 00 | 3.55E+ 01 | 0.00E+ 00 |
| DVSFRVL | 218 | 5.47301E-07 | 212.293 2 | 99.5882 7 | 0 | 124.874 | 0 |
| SSCCLAR | 219 | 5.47301E-07 | 23.3621 6 | 0 | 0 | 40.2819 3 | 0 |
| PPKSTFY | 220 | 1.09E-06 | 1.06E+ 02 | 1.18E+ 02 | 0.00E+ 00 | 8.06E+ 00 | 0.00E+ 00 |
| AWVVDL A | 221 | 5.58422E-07 | 103.891 9 | 636.363 6 | 0 | 236.878 2 | 0 |
| ICTVHRV | 222 | 5.58E-07 | 9.92E+ 01 | 0.00E+ 00 | 0.00E+ 00 | 9.08E+ 01 | 0.00E+ 00 |
| FWSFLSR | 223 | 5.58422E-07 | 27.2582 1 | 0 | 0 | 59.2195 6 | 0 |
| FPWVSWL | 224 | 5.47301E-07 | 415.607 8 | 263.128 8 | 0 | 72.5074 7 | 0 |
| LLRGIGP | 225 | 5.47E-07 | 9.90E+ 01 | 0.00E+ 00 | 0.00E+ 00 | 1. 17E+ 02 | 1.94E+ 01 |
| TETLRNP | 226 | 5.58422E-07 | 269.832 4 | 162.674 9 | 95.5330 8 | 440.528 8 | 639.033 |
| GKRIAEA | 227 | 5.58422E-07 | 112.733 4 | 85.9456 5 | 0 | 130.283 | 0 |
| KGGADLP | 228 | 5.58E-07 | 9.27E+ 01 | 2.30E+ 02 | 0.00E+ 00 | 1.42E+ 02 | 1.27E+ 01 |
| EDVPRCC | 229 | 5.58422E-07 | 224.434 8 | 160.919 5 | 0 | 102.647 2 | 788.594 8 |
| LIVHFLY | 230 | 5.58E-07 | 8.80E+ 01 | 7.46E+ 01 | 0.00E+ 00 | 7.50E+ 01 | 0.00E+ 00 |
| SESSTQT | 231 | 5.58422E-07 | 26.1678 9 | 0 | 0 | 82.9073 8 | 0 |
| NTFHNPR | 232 | 5.47301E-07 | 65.6365 5 | 14.9263 1 | 22.3106 8 | 104.733 | 451.653 |
| VIDCRSV | 233 | 5.58422E-07 | 286.558 7 | 285.266 4 | 0 | 15.7918 8 | 0 |
| WDSSLFP | 234 | 5.58422E-07 | 238.575 7 | 236.626 5 | 0 | 189.502 6 | 12.6693 4 |
| QVMPGM A | 235 | 1.11684E-06 | 40.1795 5 | 44.8014 6 | 0 | 35.5317 4 | 0 |
| RGPVRYH | 236 | 1.11E-06 | 8.12E+ 01 | 2.77E+ 01 | 0.00E+ 00 | 6.58E+ 01 | 1.17E+ 01 |
| GTVRSNQ | 237 | 2.74763E-06 | 77.5875 4 | 316.568 4 | 0 | 125.873 3 | 224.928 1 |
| LTSFDLG | 238 | 8.28E-06 | 7.59E+ 01 | 9.08E+ 01 | 2.51E+ 00 | 1.10E+ 02 | 6.32E+ 01 |
| MSRLDLN | 239 | 5.47E-07 | 7.57E+ 01 | 0.00E+ 00 | 0.00E+ 00 | 5.24E+ 01 | 0.00E+ 00 |
| PKALMIT | 240 | 2.21E-06 | 7.35E+ 01 | 0.00E+ 00 | 0.00E+ 00 | 8.00E+ 01 | 5.85E+ 00 |
| RSPCEGR | 241 | 5.58E-07 | 6.91E+ 01 | 0.00E+ 00 | 3.64E+ 01 | 1.03E+ 02 | 6.33E+ 00 |
| ISPGLFC | 242 | 5.47301E-07 | 142.704 4 | 199.176 5 | 0 | 128.902 2 | 0 |
| EANPLTS | 243 | 5.58422E-07 | 151.555 7 | 216.262 | 0 | 31.5837 7 | 16.8412 1 |
| LCSRVFS | 244 | 5.47301E-07 | 101.206 3 | 167.933 2 | 0 | 72.5074 7 | 6.46338 8 |
| LGCMCAA | 245 | 5.58422E-07 | 338.062 6 | 542.150 6 | 0 | 600.833 2 | 16.8412 1 |
| SQVNLGS | 246 | 5.58422E-07 | 71.8512 7 | 0 | 0 | 414.949 8 | 0 |
| LCHSPTS | 247 | 5.47301E-07 | 229.263 3 | 408.116 6 | 0 | 153.071 3 | 503.360 1 |
| AIPQTMQ | 248 | 5.58E-07 | 5.85E+ 01 | 0.00E+ 00 | 0.00E+ 00 | 0.00E+ 00 | 0.00E+ 00 |
| NAHVLHS | 249 | 5.47301E-07 | 132.302 6 | 246.284 1 | 0 | 145.014 9 | 12.9267 8 |
| NHTVRRR | 250 | 5.58422E-07 | 149.986 | 287.073 4 | 0 | 15.7918 8 | 0 |
| LNLRHHT | 251 | 5.58E-07 | 5.67E+ 01 | 9.51E+ 01 | 0.00E+ 00 | 8.69E+ 01 | 0.00E+ 00 |
| VTTREAQ | 252 | 5.47301E-07 | 50.0617 8 | 259.344 6 | 0 | 40.2819 3 | 0 |
| FVTREYR | 253 | 5.58E-07 | 4.71E+ 01 | 1.01E+ 02 | 0.00E+ 00 | 5.92E+ 01 | 0.00E+ 00 |
| KNKKKKA | 254 | 5.47301E-07 | 62.2991 | 149.877 6 | 0 | 36.2537 4 | 0 |
| VLLWELG | 255 | 5.58E-07 | 4.66E+ 01 | 0.00E+ 00 | 0.00E+ 00 | 0.00E+ 00 | 0.00E+ 00 |
| AMASLMA | 256 | 3.89783E-06 | 62.1654 2 | 137.64 | 15.1412 8 | 214.966 | 338.623 6 |
| VFLPGFS | 257 | 5.47301E-07 | 157.796 3 | 403.596 7 | 48.7632 5 | 84.5920 5 | 0 |
| VRQYCVI | 258 | 5.58E-07 | 4.03E+ 01 | 0.00E+ 00 | 0.00E+ 00 | 0.00E+ 00 | 0.00E+ 00 |
| PPGRIMP | 259 | 5.47E-07 | 4.00E+ 01 | 0.00E+ 00 | 0.00E+ 00 | 1.21E+ 01 | 0.00E+ 00 |
| PRVDIPT | 260 | 5.58E-07 | 3.84E+ 01 | 0.00E+ 00 | 0.00E+ 00 | 1.18E+ 01 | 0.00E+ 00 |
| VKGDNNP | 261 | 7.75118E-06 | 56.3214 6 | 137.288 4 | 6.73999 3 | 327.872 8 | 241.823 1 |
| RPDGLKL | 262 | 5.58422E-07 | 111.213 5 | 337.161 6 | 0 | 67.1155 | 6.33466 8 |
| SNTSALQ | 263 | 1.09E-06 | 3.34E+ 01 | 4.19E+ 01 | 0.00E+ 00 | 0.00E+ 00 | 1.03E+ 02 |
| **SQDHGRS** | 264 | 2.26506E-05 | 37.9232 1 | 106.436 9 | 3.19211 8 | 138.491 9 | 952.411 4 |
| VLRPEER | 265 | 1.09E-06 | 3.04E+ 01 | 0.00E+ 00 | 0.00E+ 00 | 0.00E+ 00 | 0.00E+ 00 |
| VMESVRS | 266 | 5.58422E-07 | 68.6907 | 393.155 7 | 0 | 63.1675 3 | 0 |
| RFPGAPP | 267 | 5.47301E-07 | 96.3416 2 | 591.293 3 | 0 | 84.5920 5 | 0 |
| KKTTKKK | 268 | 5.58E-07 | 2.51E+ 01 | 1.91E+ 00 | 0.00E+ 00 | 5.53E+ 01 | 6.33E+ 00 |
| IHWAGVL | 269 | 5.47301E-07 | 64.5240 7 | 618.835 6 | 0 | 36.2537 4 | 0 |
| GTCCSTR | 270 | 5.47301E-07 | 55.6241 9 | 561.325 6 | 0 | 92.6484 4 | 40.8302 4 |
| WLSSKTI | 271 | 5.47E-07 | 3.75E+ 02 | 3.91E+ 00 | 0.00E+ 00 | 9.67E+ 01 | 3.23E+ 01 |
| YKCTPPS | 272 | 5.58422E-07 | 96.8663 4 | 0 | 0 | 0 | 0 |
| HVMPQR W | 273 | 5.47E-07 | 3.42E+ 02 | 3.84E+ 02 | 0.00E+ 00 | 8.06E+ 01 | 1.72E+ 01 |
| LSYWLVL | 274 | 5.58422E-07 | 25.0775 6 | 0 | 0 | 0 | 0 |
| QDNLSGS | 275 | 1.6419E-06 | 23.4742 2 | 0 | 0 | 0 | 0 |
| PIELFGN | 276 | 5.47E-07 | 2.14E+ 02 | 3.91E+ 00 | 3.90E+ 01 | 8.86E+ 01 | 0.00E+ 00 |
| RCPNNTV | 277 | 1.11684E-06 | 77.5802 | 0 | 0 | 5.92195 6 | 0 |
| LSRFSHF | 278 | 5.58E-07 | 2.12E+ 02 | 1.76E+ 02 | 2.12E+ 01 | 7.90E+ 01 | 0.00E+ 00 |
| PQTPSCR | 279 | 5.47301E-07 | 56.0092 7 | 0 | 0 | 8.05638 6 | 0 |
| RLFYLAC | 280 | 5.47301E-07 | 24.4746 5 | 0 | 0 | 4.02819 3 | 0 |
| KLAIVHI | 281 | 5.47E-07 | 2.06E+ 02 | 2.84E+ 02 | 0.00E+ 00 | 1.29E+ 02 | 0.00E+ 00 |
| PQPTWLW | 282 | 5.47E-07 | 1.99E+ 02 | 3.11E+ 02 | 7.04E+ 01 | 2.30E+ 02 | 0.00E+ 00 |
| QTRNRWT | 283 | 5.58422E-07 | 34.8905 1 | 0 | 0 | 7.89594 1 | 0 |
| AICFCCC | 284 | 5.47301E-07 | 196.684 3 | 0 | 0 | 68.4792 8 | 0 |
| TETQFTY | 285 | 5.58E-07 | 1.83E+ 02 | 0.00E+ 00 | 0.00E+ 00 | 6.71E+ 01 | 0.00E+ 00 |
| EGKAMYS | 286 | 5.58E-07 | 1.72E+ 02 | 0.00E+ 00 | 0.00E+ 00 | 6.32E+ 01 | 0.00E+ 00 |
| EPRDMKT | 287 | 5.47E-07 | 1.70E+ 02 | 0.00E+ 00 | 0.00E+ 00 | 7.99E+ 02 | 3.66E+ 01 |
| NYWESSR | 288 | 1.10572E-06 | 48.9276 5 | 0 | 32.2091 7 | 19.9383 9 | 0 |
| KSGMAKQ | 289 | 5.47E-07 | 1.52E+ 02 | 0.00E+ 00 | 0.00E+ 00 | 2.82E+ 01 | 0.00E+ 00 |
| NLPREFG | 290 | 5.58E-07 | 1.51E+ 02 | 3.18E+ 02 | 0.00E+ 00 | 2.42E+ 02 | 1.27E+ 01 |
| NEAHHTP | 291 | 5.58E-07 | 1.49E+ 02 | 0.00E+ 00 | 0.00E+ 00 | 7.90E+ 00 | 0.00E+ 00 |
| TWPCTSY | 292 | 5.47E-07 | 1.44E+ 02 | 3.32E+ 01 | 0.00E+ 00 | 1.61E+ 01 | 0.00E+ 00 |
| GENCLLL | 293 | 5.58422E-07 | 158.623 1 | 0 | 103.865 2 | 122.387 1 | 0 |
| MTMTTQK | 294 | 5.58E-07 | 1.41E+ 02 | 7.31E+ 01 | 0.00E+ 00 | 2.37E+ 01 | 0.00E+ 00 |
| NEKSAAQ | 295 | 5.58E-07 | 1.31E+ 02 | 2.95E+ 01 | 0.00E+ 00 | 1.58E+ 01 | 0.00E+ 00 |
| NETSKQA | 296 | 3.88671E-06 | 69.9833 6 | 20.3476 2 | 6.80689 | 22.4172 2 | 36.339 |
| WKGRTM M | 297 | 5.58E-07 | 1.19E+ 02 | 0.00E+ 00 | 0.00E+ 00 | 1.46E+ 02 | 0.00E+ 00 |
| EEKLTIN | 298 | 5.58422E-07 | 211.876 3 | 0 | 0 | 106.595 2 | 819.807 |
| VNRYLFG | 299 | 5.58422E-07 | 292.493 9 | 102.573 7 | 0 | 102.647 2 | 824.286 3 |
| KPHPKTE | 300 | 5.47E-07 | 9.79E+ 01 | 2.44E+ 02 | 1.12E+ 01 | 7.65E+ 01 | 3.44E+ 01 |
| NGTIISD | 301 | 1.12E-06 | 9.57E+ 01 | 0.00E+ 00 | 0.00E+ 00 | 0.00E+ 00 | 0.00E+ 00 |
| QPGLHWR | 302 | 5.58E-07 | 9.41E+ 01 | 0.00E+ 00 | 0.00E+ 00 | 9.48E+ 01 | 0.00E+ 00 |
| YLSGVFV | 303 | 5.58422E-07 | 121.456 1 | 80.5449 5 | 0 | 207.022 5 | 0 |
| DLAKEQN | 304 | 5.58E-07 | 8.72E+ 01 | 1.19E+ 02 | 0.00E+ 00 | 9.48E+ 01 | 0.00E+ 00 |
| WFSWCVL | 305 | 5.47301E-07 | 56.7366 8 | 1.86578 8 | 0 | 181.268 7 | 17.1834 3 |
| WNGQSQS | 306 | 5.58422E-07 | 208.734 6 | 194.783 9 | 98.3986 1 | 102.647 2 | 0 |
| KSCEGML | 307 | 5.47301E-07 | 511.232 6 | 513.091 8 | 0 | 120.845 8 | 0 |
| KQRPTWR | 308 | 5.47301E-07 | 121.362 9 | 124.973 5 | 0 | 28.1973 5 | 0 |
| FVYLSYF | 309 | 5.47E-07 | 8.56E+ 01 | 0.00E+ 00 | 0.00E+ 00 | 9.26E+ 01 | 0.00E+ 00 |
| ALQCELI | 310 | 5.47E-07 | 8.45E+ 01 | 0.00E+ 00 | 1.12E+ 01 | 3.19E+ 02 | 0.00E+ 00 |
| VCLVHSQ | 311 | 5.47301E-07 | 83.4362 9 | 242.136 2 | 0 | 181.268 7 | 0 |
| LCRAYEP | 312 | 1.12E-06 | 8.16E+ 01 | 0.00E+ 00 | 0.00E+ 00 | 1.97E+ 01 | 0.00E+ 00 |
| LAGHIIK | 313 | 5.58422E-07 | 73.0520 1 | 228.578 9 | 0 | 23.6878 2 | 25.3386 7 |
| CGWVFAC | 314 | 5.58E-07 | 7.09E+ 01 | 5.38E+ 01 | 0.00E+ 00 | 6.71E+ 01 | 0.00E+ 00 |
| CWNQADD | 315 | 5.58422E-07 | 92.6779 3 | 125.034 4 | 0 | 67.1155 | 35.8452 2 |
| AILMENP | 316 | 5.47E-07 | 6.60E+ 01 | 1.44E+ 02 | 0.00E+ 00 | 1.21E+ 02 | 0.00E+ 00 |
| QMRNLAT | 317 | 5.58422E-07 | 206.879 1 | 377.549 9 | 0 | 138.179 | 0 |
| CPAFAWA | 318 | 5.58422E-07 | 163.113 7 | 296.642 5 | 0 | 144.841 3 | 6.33466 8 |
| WRPYVMP | 319 | 5.47301E-07 | 57.8491 6 | 106.349 9 | 0 | 80.5638 6 | 0 |
| KNVDAAT | 320 | 1.11684E-06 | 109.116 3 | 215.827 | 0 | 39.4797 1 | 8.42060 7 |
| ASFTGCF | 321 | 1.12E-06 | 4.66E+ 01 | 9.78E+ 01 | 0.00E+ 00 | 6.32E+ 01 | 0.00E+ 00 |
| LREYDTT | 322 | 5.47E-07 | 4.56E+ 01 | 0.00E+ 00 | 0.00E+ 00 | 3.64E+ 02 | 1.29E+ 01 |
| NHANKKS | 323 | 5.58422E-07 | 47.7972 9 | 129.832 8 | 0 | 82.9073 8 | 0 |
| AFITRSF | 324 | 5.58E-07 | 3.71E+ 01 | 0.00E+ 00 | 0.00E+ 00 | 1.18E+ 01 | 0.00E+ 00 |
| QNSEKRM | 325 | 1.11684E-06 | 34.3453 5 | 103.317 6 | 0 | 5.92195 6 | 0 |
| FFFFVVC | 326 | 5.58422E-07 | 29.4388 7 | 0 | 0 | 395.209 9 | 0 |
| ENKKKKH | 327 | 5.58E-07 | 3.05E+ 01 | 0.00E+ 00 | 0.00E+ 00 | 1.58E+ 01 | 0.00E+ 00 |
| IVYGFSC | 328 | 5.47E-07 | 4.52E+ 02 | 6.09E+ 02 | 0.00E+ 00 | 2.38E+ 02 | 0.00E+ 00 |
| FPWWPGQ | 329 | 5.47E-07 | 3.74E+ 02 | 1.08E+ 02 | 0.00E+ 00 | 3.63E+ 01 | 3.44E+ 01 |
| EQDSSAL | 330 | 1.6419E-06 | 54.1324 3 | 0 | 0 | 0 | 0 |
| LLYPCEM | 331 | 5.58E-07 | 2.89E+ 02 | 4.21E+ 01 | 0.00E+ 00 | 9.87E+ 01 | 0.00E+ 00 |
| KNVPWLR | 332 | 5.47E-07 | 2.64E+ 02 | 5.47E+ 01 | 0.00E+ 00 | 1.57E+ 02 | 2.36E+ 01 |
| CGYTFLR | 333 | 1.12E-06 | 2.46E+ 02 | 0.00E+ 00 | 0.00E+ 00 | 1.97E+ 01 | 0.00E+ 00 |
| KVDPVIG | 334 | 1.65302E-06 | 39.8814 5 | 0 | 0 | 0 | 0 |
| IYIDTTG | 335 | 5.47301E-07 | 189.228 8 | 0 | 0 | 8.05638 6 | 0 |
| TMTEPIQ | 336 | 5.58E-07 | 2.14E+ 02 | 5.10E+ 02 | 4.01E+ 01 | 3.55E+ 01 | 0.00E+ 00 |
| FCKFRLE | 337 | 5.47E-07 | 2.13E+ 02 | 2.34E+ 02 | 0.00E+ 00 | 6.04E+ 01 | 1.72E+ 01 |
| SHIDGPE | 338 | 5.47E-07 | 1.96E+02 | 2.38E+02 | 0.00E+00 | 7.65E+01 | 4.93E+02 |
| FVLCEWE | 339 | 5.47E-07 | 1.48E+ 02 | 0.00E+ 00 | 0.00E+ 00 | 2.01E+ 01 | 0.00E+ 00 |
| RFHPIAH | 340 | 5.58E-07 | 1.31E+ 02 | 0.00E+ 00 | 0.00E+ 00 | 0.00E+ 00 | 0.00E+ 00 |
| VLNSDCF | 341 | 5.47E-07 | 1.11E+ 02 | 0.00E+ 00 | 0.00E+ 00 | 0.00E+ 00 | 0.00E+ 00 |
| LSDAFFK | 342 | 5.58E-07 | 1.07E+ 02 | 0.00E+ 00 | 0.00E+ 00 | 3.95E+ 00 | 0.00E+ 00 |
| NEGMPFC | 343 | 5.58422E-07 | 244.456 9 | 208.463 9 | 0 | 63.1675 3 | 0 |
| RWVGQSK | 344 | 5.47E-07 | 8.97E+ 01 | 0.00E+ 00 | 0.00E+ 00 | 0.00E+ 00 | 0.00E+ 00 |
| YQQDNVH | 345 | 5.58E-07 | 8.71E+ 01 | 1.13E+ 02 | 0.00E+ 00 | 2.01E+ 02 | 3.80E+ 01 |
| CMIEDVD | 346 | 5.47E-07 | 6.28E+ 01 | 0.00E+ 00 | 0.00E+ 00 | 1.21E+ 01 | 0.00E+ 00 |
| RGGFQIF | 347 | 5.47E-07 | 5.69E+ 01 | 0.00E+ 00 | 0.00E+ 00 | 2.82E+ 01 | 0.00E+ 00 |
| QQETLYP | 348 | 3.86447E-06 | 29.8356 2 | 88.4961 3 | 6.05613 7 | 112.992 7 | 19.4908 7 |
| RMITIEP | 349 | 1.09E-06 | 3.17E+ 01 | 1.27E+ 01 | 8.37E+ 00 | 5.44E+ 01 | 3.23E+ 00 |
| QKHSMLE | 350 | 5.58422E-07 | 28.3485 4 | 128.226 1 | 0 | 15.7918 8 | 25.3386 7 |
| IQYMSHQ | 351 | 5.47E-07 | 2.43E+ 02 | 0.00E+ 00 | 0.00E+ 00 | 8.46E+ 01 | 0.00E+ 00 |
| TMLEDMI | 352 | 5.47E-07 | 2.23E+ 02 | 4.69E+ 02 | 0.00E+ 00 | 3.42E+ 02 | 1.29E+ 01 |
| RQEKQNK | 353 | 5.58E-07 | 1.87E+ 02 | 0.00E+ 00 | 0.00E+ 00 | 6.71E+ 01 | 1.27E+ 01 |
| YMETWDF | 354 | 5.47E-07 | 1.38E+ 02 | 0.00E+ 00 | 0.00E+ 00 | 0.00E+ 00 | 0.00E+ 00 |
| CCACYQA | 355 | 1.09E-06 | 9.86E+ 01 | 0.00E+ 00 | 0.00E+ 00 | 4.23E+ 01 | 0.00E+ 00 |
| YRFCAQI | 356 | 5.47E-07 | 7.01E+ 01 | 0.00E+ 00 | 0.00E+ 00 | 2.82E+ 01 | 1.72E+ 01 |
| YQCEHQA | 357 | 5.58E-07 | 2.40E+ 02 | 4.42E+ 02 | 0.00E+ 00 | 3.16E+ 01 | 0.00E+ 00 |
| >AAV1 | | 1.47E-03 | 2.33E+ 01 | 3.35E+ 01 | 6.62E+ 02 | 1.15E+ 02 | 2.84E+ 02 |

### Experimental Example 12: Preparation of AAV1-based adeno-associated virus variants

A DNA sequence corresponding to a 7-mer peptide selected in the same manner as in Experimental Example 10 was inserted into a recombinant AAV plasmid vector pAAV2/1 (Adgene) consisting of the wild-type AAV1 capsid gene and the wild-type AAV2 rep gene. In addition, pAAV2/1.p2CV was prepared by substituting the AAV1.p2CV capsid DNA from pSub2-AAV1.p2CV obtained in Experimental Example 10 into the wild-type AAV1 capsid gene position of pAAV2/1, and a DNA sequence corresponding to a 7-mer peptide selected in the same manner as in Experimental Example 10 was inserted. A recombinant variant in which the SPNSAGV sequence was inserted between the amino acids at positions 588 and 589 in the capsid amino acid sequence of the wild-type AAV1 in this manner was named AAV1.SP, and the corresponding plasmid was named pAAV2/1.SP. Additionally, a recombinant variant in which the SGVSILG sequence was inserted was named AAV1.SG, and the corresponding plasmid was named pAAV2/1.SG. Furthermore, a recombinant variant in which the SPNSAGV sequence was inserted into the AAV1.p2CV in addition to the AAV1.SP was named AAV1.p2CV.SP, and the corresponding plasmid was named pAAV2/1.p2CV.SP. In addition, a recombinant variant in which the SGVSILG sequence was inserted into the AAV1.p2CV as in the AAV1.SG was named AAV1.p2CV.SG, and the corresponding plasmid was named pAAV2/1.p2CV.SG.

To load the human FGF12 (hFGF12) gene, a pulmonary arterial hypertension treatment gene, into the AAV1-based adeno-associated virus variant prepared by the method described above, the plasmids pAAV2/1.p2CV, pAAV2/1.SP, pAAV2/1.p2CV.SP, pAAV2/1.SG, or pAAV2/1.p2CV.SG, the ITR-flanked hFGF12 gene (pCMV-hFGF12), and pHelper were complexed using PEIpro (Polyplus, Cat No.: 101000033) and transfected into HEK293T cells. After about 72 hours, only the cell pellets were collected and the AAV inside the cells was extracted by freezing and thawing. Subsequently, cell debris was removed by centrifugation, and nucleic acids were removed from virus-producing cells by incubating with Benzonase 10 U/mL at 37 °C for 30 minutes to obtain an AAV solution. The AAV solution was purified by iodixanol density gradient ultracentrifugation (OptiPrep, Alere Technologies AS, Oslo, Norway) with a Vti65.2 rotor (Beckman Coulter; 42,000 rpm). Recombinant variants AAV1.p2CV, AAV1.SP, AAV1.p2CV.SP, AAV1.SG, and AAV1.p2CV.SG packaged with the therapeutic gene hFGF12 were obtained by buffer-exchanging the purified AAV vector with a 1x PBS solution containing 0.01% (v/v) Tween 20 (Sigma-Aldrich) using an Amicon tube (Merck Millipore, Billerica, MA; molecular weight cutoff: 100 kDa).

### Experimental Example 13: Comparison of delivery and expression capabilities of wild-type AAV1 and AAV1.p2CV on lung cells in mouse model of pulmonary arterial hypertension

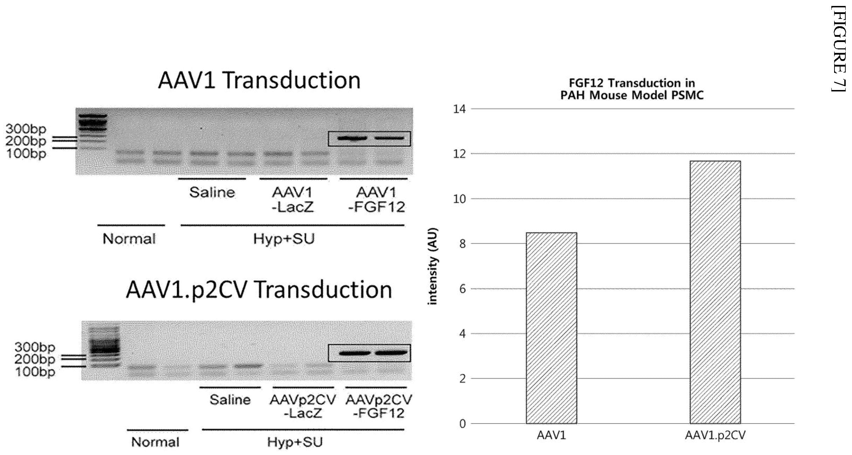

Lung airway aerosol administration was performed in the same manner as in Experimental Example 11 on a mouse model of pulmonary arterial hypertension induced according to Experimental Example 9 by packaging the hFGF12 gene into the wild-type AAV1 and the AAV1.p2CV variant prepared in Experimental Example 12. Two weeks after the administration, mRNA was isolated from lung tissues obtained by necropsy, and reverse transcription polymerase chain reaction (RT-PCR) was performed on the hFGF12 gene delivered via AAV. The results and ImageJ analysis are shown in FIG. 7. It can be seen that the AAV1.p2CV variant exhibits a higher hFGF12 mRNA expression level in lung tissues than the wild-type AAV1.

### Experimental Example 14: Experiment on delivery and expression of AAV variants in human pulmonary artery smooth muscle cells (hPASMCs)

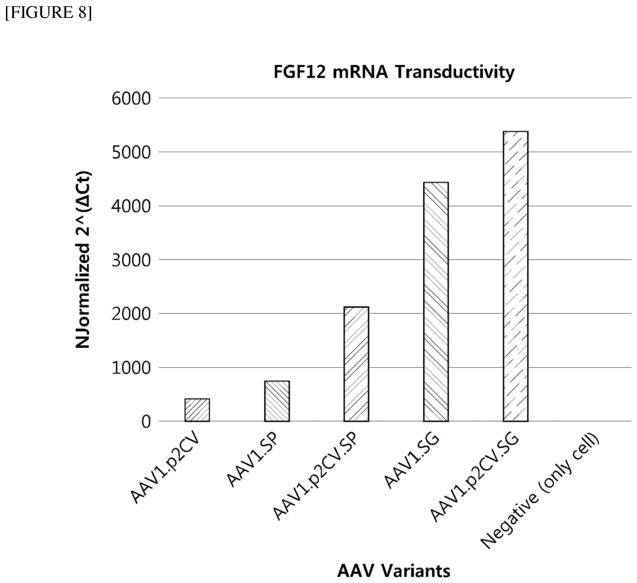

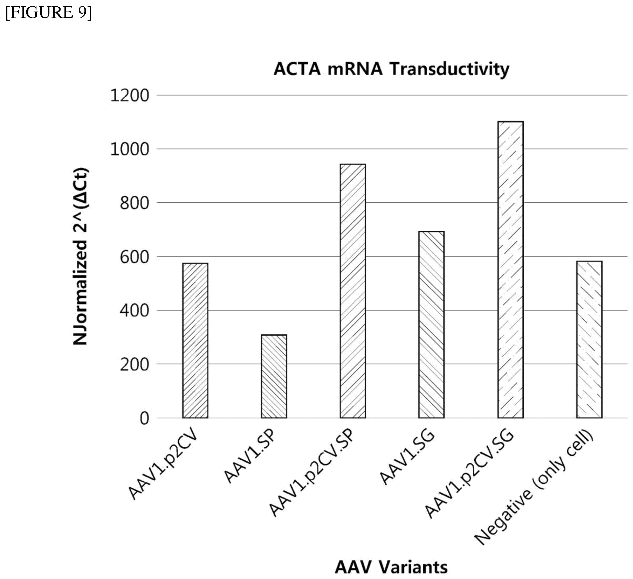

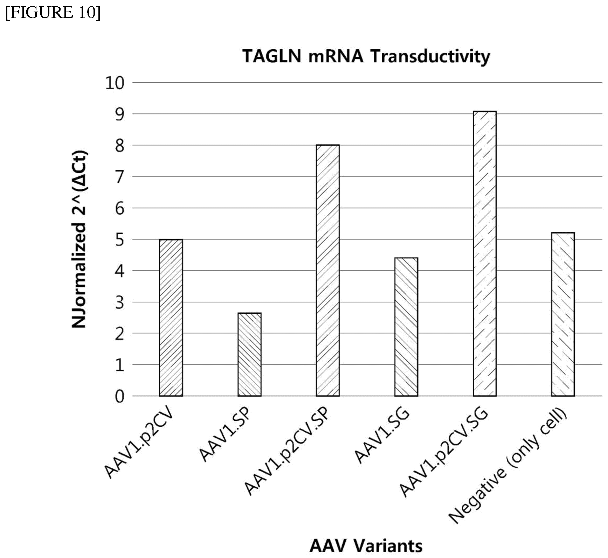

An *in vitro* experiment was performed on the delivery of the hFGF12 gene into human pulmonary artery smooth muscle cells and mRNA expression for the recombinant AAV variants AAV1.p2CV, AAV1.SP, AAV1.p2CV.SP, AAV1.SG, and AAV1.p2CV.SG packaged with the therapeutic gene hFGF12, which were prepared in Experimental Example 12. In the experiment, human pulmonary artery smooth muscle cells (hPASMC, American Type Culture Collection (ATCC); 2x10⁴ cells/20 µL) were infected with the recombinant AAV variants packaged with hFGF12 at an MOI of 100,000. Three days later, mRNA was isolated from the hPASMCs using the RNeasy Plus kit (Qiagen), and complementary DNA (cDNA) was obtained. From the obtained cDNA, the hFGF12 gene and the ACTA and TAGLN genes expressed by it were extracted. The cDNA level of each gene was measured by qPCR, and normalized to the qPCR result of cDNA of the GAPDH mRNA expression. The experimental result for the respective genes is shown in FIGS. 8 to 10.

Based on the experimental result shown in FIGS. 8 to 10, it was confirmed that AAV1-based point mutations (preferably point mutations of the p2CV variant) and mutations involving the insertion of a 7-mer peptide (preferably SPNSAGV or SGVSILG) between the 588th and 589th amino acid positions exhibited a synergistic effect of significantly enhancing specificity for human pulmonary artery smooth muscle cells. Meanwhile, the p2CV variant had excellent diffusivity, allowing for gene delivery to pulmonary artery smooth muscle cells via airway aerosol administration (refer to Korean Patent Application No. 10-2022-0120442). However, according to Experimental Example 8, the target specificity for human pulmonary artery smooth muscle cells was slightly higher than that of the wild-type AAV1. On the other hand, when the 7-mer peptide was inserted into the wild-type AAV1, it showed a higher hFGF12 expression level than p2CV. In addition, when the 7-mer peptide insertion mutation was combined with the p2CV point mutation, the specificity for human pulmonary artery smooth muscle cells was unexpectedly and significantly improved as compared to when it was inserted into the wild-type AAV1. These experimental results support the synergistic effect of the combination. Furthermore, according to Experimental Example 13, AAV1.p2CV showed higher hFGF12 expression than the wild-type AAV1 in a mouse model of pulmonary arterial hypertension. Considering the synergistic effect of the combination, it can be seen that the combination of the point mutation shown in Table 1 and the 7-mer insertion peptide shown in Table 3 can significantly increase target specificity for lung smooth muscle cells.

### Experimental Example 15: Experiment on preventive effect for pulmonary arterial hypertension

Using the recombinant AAV1 and the recombinant variants AAV1.p2CV, AAV1.p2CV.SP, and AAV1.p2CV.SG packaged with the therapeutic gene hFGF12 prepared as described in Experimental Example 12, the preventive effect against pulmonary arterial hypertension (PAH) was tested *in vivo* in the mouse model prepared in Experimental Example 9. Each of the AAV carriers, namely the wild-type recombinant AAV1 and the AAV1.p2CV, AAV1.p2CV.SP, and AAV1.p2CV.SG packaged with hFGF12, was administered in the form of an aerosol into the airways of mice using a PenWu microaerosolizer (BioJane, Shanghai, China) (1.25" length of intratracheal portion, 700 µm outer diameter, 430 µm inner diameter), after which pulmonary arterial hypertension was induced under a Su5416 + hypoxic environment.

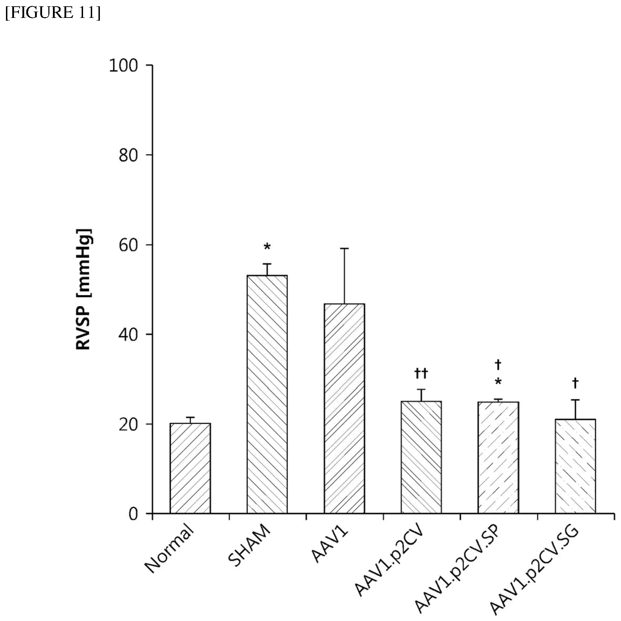

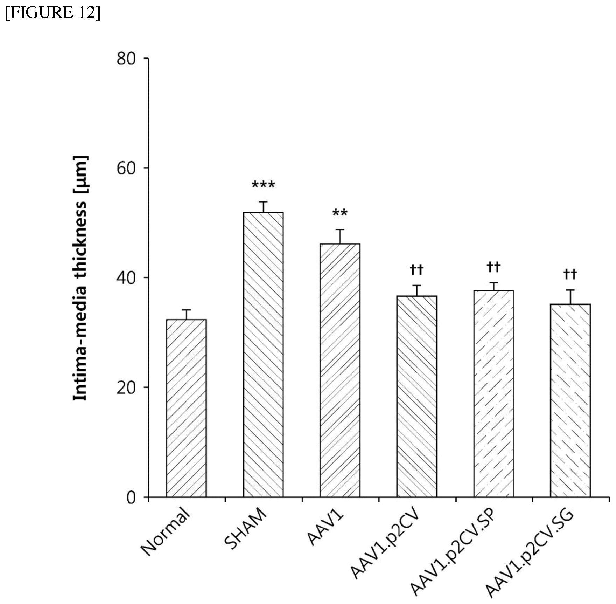

FIG. 11 shows the result of measuring right ventricular systolic pressure (RVSP), and FIG. 12 shows the result of measuring intima-media thickness. Normal represents normal mice, and SHAM represents PAH mice that underwent airway management surgery without virus injection (t-test for Normal: * > 0.05, ** > 0.01, *** > 0.001; t-test for SHAM: ^{†} > 0.05, ^{††} > 0.01, ^{†††} > 0.001). AAV1.p2CV is a p2CV variant of the wild-type AAV1 containing two point mutations, S430C and I647V, and variants combined with SPNSAGV or SGVSILG peptide insertion were named AAV1.p2CV.SP or AAV1.p2CV.SG, respectively.

As shown in FIG. 11 and FIG. 12, it was confirmed that the wild-type AAV1-based point mutation or a 7-mer insertion mutation combined with it significantly improves the preventive effect against pulmonary arterial hypertension, with the vascular thickness value remaining close to the normal value.

## Claims

1. A variant of the AAV1 (adeno-associated virus serotype 1) capsid protein, comprising,
as compared to the amino acid sequence of the wild-type AAV1 capsid protein represented by SEQ ID NO: 1,
one or more amino acid substitutions selected from the group consisting of substitution of asparagine (N) at the 14th position with threonine (T) (N14T), substitution of glutamic acid (E) at the 21st position with glutamine (Q) (E21Q), substitution of tryptophan (W) at the 23rd position with arginine (R) (W23R), substitution of aspartic acid (D) at the 24th position with lysine (K) (D24K), substitution of lysine (K) at the 31st position with threonine (T) (K31T), substitution of proline (P) at the 32nd position with threonine (T) (P32T), substitution of asparagine (N) at the 35th position with arginine (R) (N35R), substitution of glutamine (Q) at the 36th position with glutamic acid (E) (Q36E), substitution of glutamine (Q) at the 37th position with arginine (R) (Q37R), substitution of lysine (K) at the 38th position with histidine (H) (K38H), substitution of glutamine (Q) at the 39th position with lysine (K) (Q39K), substitution of glycine (G) at the 42nd position with serine (S) (G42S), substitution of lysine (K) at the 137th position with glutamic acid (E) (K137E), substitution of lysine (K) at the 310th position with arginine (R) (K310R), substitution of threonine (T) at the 415th position with isoleucine (I) (T415I), substitution of serine (S) at the 430th position with arginine (R) (S430R),
substitution of serine (S) at the 430th position with cysteine (C) (S430C), substitution of serine (S) at the 430th position with glutamic acid (E) (S430E), substitution of threonine (T) at the 492nd position with alanine (A) (T492A), substitution of serine (S) at the 516th position with threonine (T) (S516T), substitution of asparagine (N) at the 583rd position with aspartic acid (D) (N583D), substitution of isoleucine (I) at the 647th position with alanine (A) (I647A), substitution of isoleucine (I) at the 647th position with valine (V) (I647V), substitution of isoleucine (I) at the 647th position with tyrosine (Y) (I647Y), substitution of alanine (A) at the 660th position with valine (V) (A660V), and substitution of glutamine (Q) at the 688th position with arginine (R) (Q688R), and comprising
an insertion sequence of X₁X₂X₃X₄X₅X₆X₇ (Formula I).

2. The variant of the AAV1 capsid protein according to claim 1, wherein,
as compared to the wild-type AAV1 capsid protein, the variant of the AAV1 capsid protein
comprises the S430C and I647V substitutions;
comprises the S516T, N583D and A660V substitutions;
comprises the K310R substitution;
comprises the W23R and T415I substitutions;
comprises the N14T, E21Q, D24K, K31T, P32T, N35R, Q36E, Q37R, K38H, Q39K and G42S substitutions;
comprises the Q688R substitution;
comprises the T492A substitution;
comprises the K137E substitution;
comprises the S430R substitution;
comprises the S430E substitution;
comprises the I647A substitution; or
comprises the I647Y substitution.

3. The variant of the AAV1 capsid protein according to claim 1, wherein the insertion sequence is inserted between the amino acids at the 588th and 589th positions of the amino acid sequence of the wild-type AAV1 (SEQ ID NO: 1).

4. The variant of the AAV1 capsid protein according to claim 1, wherein
the amino acid X₁ is any one selected from the group consisting of S, L, A, P, N, V, D, R, E, Q and T,
the amino acid X₂ is any one selected from the group consisting of S, N, G, R, T, A, P, E, V, Q and K,
the amino acid X₃ is any one selected from the group consisting of T, E, S, N, V, P, R, A, G, K and D,
the amino acid X₄ is any one selected from the group consisting of S, T, P, K, D, Q, Y, A, V, L and N,
the amino acid X₅ is any one selected from the group consisting of P, S, T, D, G, A, N, L, K, R and I,
the amino acid X₆ is any one selected from the group consisting of L, Q, T, V, N, G, P, S, A, K and D, or
the amino acid X₇ is any one selected from the group consisting of S, T, P, G, Q, K, A, R, V, H and E.

5. The variant of the AAV1 capsid protein according to claim 1, wherein the insertion sequence is any one selected from the group consisting of SGVSILG, SPNSAGV and SQDHGRS.

6. The variant of the AAV1 capsid protein according to claim 1, wherein the variant of the AAV1 capsid protein has tropism for smooth muscle cells.

7. The variant of the AAV1 capsid protein according to claim 1, wherein the variant of the AAV1 capsid protein comprises the S430C and I647V substitutions as compared to the wild-type AAV1 capsid protein, and the insertion sequence is SPNSAGV.

8. The variant of the AAV1 capsid protein according to claim 1, wherein the variant of the AAV1 capsid protein comprises the S430C and I647V substitutions as compared to the wild-type AAV1 capsid protein, and the insertion sequence is SGVSILG.

9. A nucleic acid comprising a nucleotide sequence represented by SEQ ID NO: 25, encoding the variant of the AAV1 capsid protein according to claim 7.

10. A nucleic acid comprising a nucleotide sequence represented by SEQ ID NO: 26, encoding the variant of the AAV1 capsid protein according to claim 8.

11. A recombinant AAV1 vector comprising the nucleic acid encoding the variant of the AAV1 capsid protein according to claim 9.

12. A recombinant AAV1 vector comprising the nucleic acid encoding the variant of the AAV1 capsid protein according to claim 10.

13. The recombinant AAV vector according to claim 11 or 12, wherein the recombinant AAV1 vector has an improved transduction profile for smooth muscle cells as compared to the AAV1 wild-type virus vector.

14. A pharmaceutical composition for preventing or treating a smooth muscle cell-related disease, comprising the recombinant AAV1 vector according to claim 11 or 12.

15. The pharmaceutical composition according to claim 14, wherein the smooth muscle cell-related disease is pulmonary arterial hypertension.

16. A variant of the AAV1 (adeno-associated virus serotype 1) capsid protein, comprising,
as compared to the amino acid sequence of the wild-type AAV1 capsid protein represented by SEQ ID NO: 1,
one or more amino acid substitutions selected from the group consisting of
substitution of asparagine (N) at the 14th position with threonine (T) (N14T),
substitution of glutamic acid (E) at the 21st position with glutamine (Q) (E21Q),
substitution of tryptophan (W) at the 23rd position with arginine (R) (W23R),
substitution of aspartic acid (D) at the 24th position with lysine (K) (D24K),
substitution of lysine (K) at the 31st position with threonine (T) (K31T),
substitution of proline (P) at the 32nd position with threonine (T) (P32T),
substitution of asparagine (N) at the 35th position with arginine (R) (N35R),
substitution of glutamine (Q) at the 36th position with glutamic acid (E) (Q36E),
substitution of glutamine (Q) at the 37th position with arginine (R) (Q37R),
substitution of lysine (K) at the 38th position with histidine (H) (K38H),
substitution of glutamine (Q) at the 39th position with lysine (K) (Q39K),
substitution of glycine (G) at the 42nd position with serine (S) (G42S),
substitution of lysine (K) at the 137th position with glutamic acid (E) (K137E),
substitution of lysine (K) at the 310th position with arginine (R) (K310R),
substitution of threonine (T) at the 415th position with isoleucine (I) (T415I),
substitution of serine (S) at the 430th position with arginine (R) (S430R),
substitution of serine (S) at the 430th position with cysteine (C) (S430C),
substitution of serine (S) at the 430th position with glutamic acid (E) (S430E),
substitution of threonine (T) at the 492nd position with alanine (A) (T492A),
substitution of serine (S) at the 516th position with threonine (T) (S516T),
substitution of asparagine (N) at the 583rd position with aspartic acid (D) (N583D),
substitution of isoleucine (I) at the 647th position with alanine (A) (I647A),
substitution of isoleucine (I) at the 647th position with valine (V) (I647V),
substitution of isoleucine (I) at the 647th position with tyrosine (Y) (I647Y),
substitution of alanine (A) at the 660th position with valine (V) (A660V), and
substitution of glutamine (Q) at the 688th position with arginine (R) (Q688R).
